# FASCICULE DE BREVET EUROPEEN

(11) **EP 0 435 776 B1**
(45) Date de publication et mention de la délivrance du brevet: **13.09.1995**
(21) Numéro de dépôt: 90403797.5
(22) Date de dépôt: 28.12.1990
(51) Int. Cl.: C12N 15/81, C12N 15/53, C12N 15/62

(54) **Promoteur artificiel pour l'expression de protéines dans la levure**
Künstlicher Promoter zur Proteine Expression in Hefe
Artificial promoter for protein expression in yeast

(30) Priorité: 29.12.1989 FR 8917467
(43) Date de publication de la demande: 03.07.1991
(73) Titulaire: SANOFI, 75008 Paris (FR)
(72) Inventeur: Leplatois, Pascal, F-81470 Cuq Toulza (FR); Loison, Gérard, F-31300 Toulouse (FR); Pessegue, Bernard, Labastidette, F-31600 Muret (FR); Shire, David, F-31520 Ramonville St Agne (FR)
(74) Mandataire: Gillard, Marie-Louise

(56) Documents cités:
- EP-A- 0 164 556
- EP-A- 0 174 585
- EP-A- 0 273 800
- EP-A- 0 284 044
- US-A- 3 810 820
- J. MOL. BIOL. vol. 186, 1985, pages 821-824, London, GB; Y. LORCH et al.:
- PATENT ABSTRACTS OF JAPAN vol. 13, no. 238 (C-603)(3586), 5 juin 1989; & JP - A - 151089 (YAMASA SHOYU CO. LTD.) 27.02.1989
- MOLECULAR AND CELLULAR BIOLOGY vol. 6, no. 1, janvier 1986, pages 246-256, Washington DC,US; M. TAJIMA et al.:
- THE JOURNAL OF BIOLOGICAL CHEMISTRY vol. 258, no. 4, 25 février 1983, pages 2674-2682, Baltimore, US; D.W. RUSSELL et al.:

## Description

La présente invention concerne un nouveau promoteur artificiel pour l'expression dans la levure de protéines, en particulier des protéines hétérologues, un vecteur d'expression de celles-là portant celui-ci, les souches de levure et notamment de Saccharomyces cerevisiae transformées par ce vecteur d'expression, ainsi qu'un procédé de production d'une protéine recombinante à l'aide de ces souches.

La levure, et en particulier Saccharomyces cerevisiae, microorganisme non pathogène dont la génétique a été étudiée en détail, est un hôte eucaryote de choix pour l'expression des protéines, notamment des protéines hétérologues. Il est donc important de découvrir ou de construire de nouveaux promoteurs pour l'expression de celles-là, plus intéressants que les promoteurs connus.

La structure d'un promoteur de levure, séquence d'ADN située en amont d'un gène et responsable de la transcription de celui-ci, commence à être partiellement connue et comprise. On sait qu'il comporte un élément TATA situé dans une zone riche en AT, une région d'initiation de la transcription en aval de celui-ci et éventuellement en amont de ce dernier des séquences, dites séquences d'activation amont UAS (Upstream Activating Sequence) ou de répression amont URS (Upstream Repressing Sequence), qui règlent la force du promoteur sous l'effet d'un inducteur ou d'un répresseur.

La demanderesse a construit un nouveau promoteur hybride à partir de deux promoteurs connus: le promoteur du gène GAL7 de Saccharomyces cerevisiae (TAJIMA et al., 1986, Molecular cellular Biology, 6, 246-256) et un promoteur de séquence proche de celle du promoteur ADH₂ naturel (région 5' flanquante du gène ADH₂ décrite par RUSSEL et al. (1983), J. Biol. Chem. 258, 2674-2682) appelé dans cette demande, un promoteur ADH₂.

L'invention concerne un nouveau promoteur artificiel pour l'expression de protéines dans la levure, caractérisé en ce qu'il comporte :
- une sous-séquence en amont de l'élément TATA de la séquence du promoteur du gène GAL7 de Saccharomyces cerevisiae, qui comporte les séquences d'activation amont UAS1 et UAS2.
- une sous-séquence de la séquence d'un promoteur ADH₂ comprenant l'élément TATA et la région d'initiation de la transcription.

De préférence, la sous-séquence en amont de l'élément TATA du promoteur du gène GAL7 de Saccharomyces cerevisiae est la séquence suivante ou une sous-séquence de celle-ci :

M
l
u
I
UAS1
CGCGTCTATACTTCGGAGCACTGTTGAGCGAAGGCTCATTAGATATATTTTCTGTCAT
--♦---------♦---------♦---------♦---------♦---------♦-----
AGATATGAAGCCTCGTGACAACTCGCTTCCGAGTAATCTATATAAAAGACAGTA
UAS2
TTTCCTTAACCCAAAAATAAGGGAGAGGGTCCAAAAAGCGCTCGGACAACTGTTGACCGT
----♦---------♦---------♦---------♦---------♦---------♦-----
AAAGGAATTGGGTTTTTATTCCCTCTCCCAGGTTTTTCGCGAGCCTGTTGACAACTGGCA
GATCCGAAGGACTGGCTATACAGTGTTCACAAAATAGCCAAGCTGAAAATAATGTGTAGC
----♦---------♦---------♦---------♦---------♦---------♦-----
CTAGGCTTCCTGACCGATATGTCACAAGTGTTTTATCGGTTCGACTTTTATTACACATCG
S
p
h
I
CTTTAGCTATGTTCAGTTAGTTTGGCATG
----♦---------♦---------♦-----
GAAATCGATACAAGTCAATCAAACC

On apprécie que la sous-séquence de la séquence d'un promoteur ADH₂ comprenant l'élément TATA et la région d'initiation de la transcription soit la séquence ci-aprés ou une sous-séquence de celle-ci :

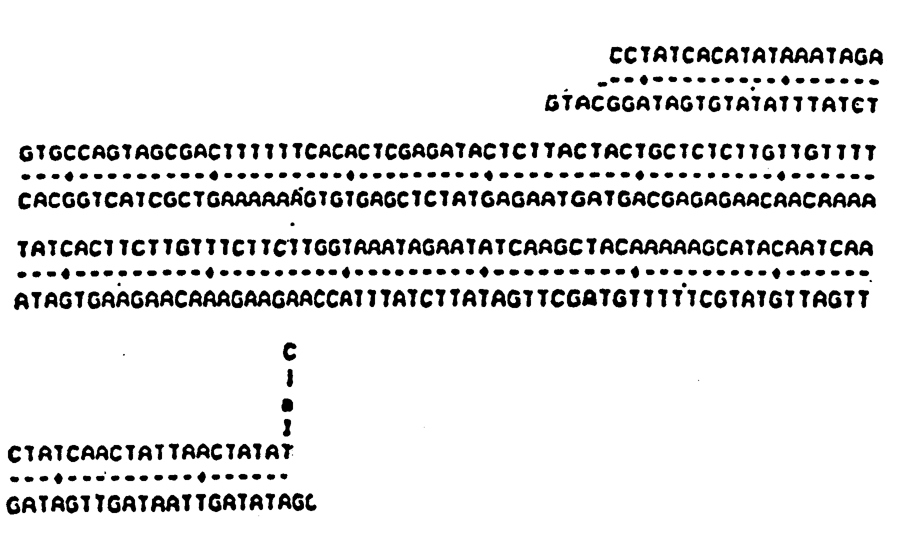
CCTATCACATATAAATAGA
GTACGGATAGTGTATATTTATCT

GTGCCAGTAGCGACTTTTTTCACACTCGAGATACTCTTACTACTGCTCTCTTGTTGTTTT
CACGGTCATCGCTGAAAAAAGTGTGAGCTCTATGAGAATGATGACGAGAGAACAACAAAA

TATCACTTCTTGTTTCTTCTTGGTAAATAGAATATCAAGCTACAAAAAGCATACAATCAA
ATAGTGAAGAACAAAGAAGAACCATTTATCTTATAGTTCGATGTTTTTCGTATGTTAGTT

C
l
a
I
CTATCAACTATTAACTATAT
GATAGTTGATAATTGATATAGC

Un promoteur particulièrement intéressant est celui qui comprend la séquence suivante :

M
l
u
I
CGCGTCTATACTTCGGAGCACTGTTGAGCGAAGGCTCATTAGATATATTTTCTGTCAT
--+---------+---------+---------+---------+---------+-----
AGATATGAAGCCTCGTGACAACTCGCTTCCGAGTAATCTATATAAAAGACAGTA

TTTCCTTAACCCAAAAATAAGGGAGAGGGTCCAAAAAGCGCTCGGACAACTGTTGACCGT
----+---------+---------+---------+---------+---------+-----
AAAGGAATTGGGTTTTTATTCCCTCTCCCAGGTTTTTCGCGAGCCTGTTGACAACTGGCA

GATCCGAAGGACTGGCTATACAGTGTTCACAAAATAGCCAAGCTGAAAATAATGTGTAGC
----+---------+---------+---------+---------+---------+-----
CTAGGCTTCCTGACCGATATGTCACAAGTGTTTTATCGGTTCGACTTTTATTACACATCG

S
p
h
I
CTTTAGCTATGTTCAGTTAGTTTGGCATGCCTATCACATATAAATAGA
----+---------+---------+---------+---------+---
GAAATCGATACAAGTCAATCAAACCGTACGGATAGTGTATATTTATCT

GTGCCAGTAGCGACTTTTTTCACACTCGAGATACTCTTACTACTGCTCTCTTGTTGTTTT
---+---------+---------+---------+---------+---------+------
CACGGTCATCGCTGAAAAAAGTGTGAGCTCTATGAGAATGATGACGAGAGAACAACAAAA

TATCACTTCTTGTTTCTTCTTGGTAAATAGAATATCAAGCTACAAAAAGCATACAATCAA
---+---------+---------+---------+---------+---------+------
ATAGTGAAGAACAAAGAAGAACCATTTATCTTATAGTTCGATGTTTTTCGTATGTTAGTT

C
l
a
I
CTATCAACTATTAACTATAT
---+---------+------
GATAGTTGATAATTGATATAGC

Le promoteur selon l'invention peut être obtenu par les techniques classiques d'ADN recombinant bien connues de l'homme de l'art.

Le promoteur de l'invention présente des avantages importants par rapport aux promoteurs connus et en particulier par rapport au promoteur ADH₂ et celui du gène GAL7.

Il permet un niveau maximal de transcription et donc d'expression élevée, en particulier pour l'urate oxydase d'A. flavus, et offre la possibilité de réguler celui-là à trois niveaux:
- en présence de glucose et en absence de galactose niveau nul: on ne détecte pas d'expression, résultat identique à celui publié pour le promoteur du gène GAL7 dans une souche de Saccharomyces cerevisiae poussant dans ces conditions (TAJIMA et al., 1986, Molecular cellular Biology, 6, 246-256). Le promoteur ADH₂ présente un niveau d'expression faible mais détectable dans ces conditions.
- en l'absence de glucose et de galactose niveau de fond: il y a une expression faible, résultat intermédiaire entre celui observé pour le promoteur ADH₂ (niveau maximal) et celui publié pour le promoteur du gène GAL7 (niveau nul: TAJIMA et al., 1986, Molecular cellular Biology, 6, 246-256).
- en l'absence de glucose et en présence de galactose niveau d'expression maximal.

L'intérêt, pour l'expression de protéines hétérologues dans la levure, de disposer d'un promoteur présentant un niveau nul dans certaines conditions est d'avoir la possibilité d'éviter toute pression de sélection qui favoriserait les cellules les moins productrices pendant la propagation de la souche. Ceci est particulièrement important dans le cas où la protéine est toxique pour la cellule hôte.

L'avantage d'un promoteur à deux niveaux d'expression: l'un de fond (non induit) et l'autre maximal (induit) réside dans la faculté de choisir un niveau intermédiaire en jouant sur la concentration de l'inducteur.

L'invention a également trait à un vecteur d'expression pour la levure portant un gène d'intérêt avec les moyens nécessaires à son expression, sa réplication et la sélection des cellules transformées, caractérisé en ce que le gène d'intérêt est sous le contrôle du promoteur précédemment défini.

Ce gène d'intérêt peut être un gène endogène de la levure ou un gène exogène eucaryote ou procaryote. Comme gène exogène eucaryote on apprécie particulièrement un gène recombinant codant pour l'urate oxydase d'Aspergillus flavus, un gène recombinant codant pour une cytokine humaine ainsi qu'un gène recombinant codant pour l'hirudine.

Dans le cas où la protéine codée par le gène exogène est sécrétée naturellement, la séquence codant pour cette protéine est de préférence précédée d'une séquence signal. Cette séquence signal, choisie en fonction de la cellule hôte, a pour fonction de permettre l'exportation de la protéine recombinante en dehors du cytoplasme, ce qui permet à la protéine recombinante de prendre une conformation proche de celle de la protéine naturelle et facilite considérablement sa purification. Cette séquence signal peut être clivée, soit en une seule étape par une signal-peptidase qui libère la protéine mature, la séquence éliminée étant habituellement appelée séquence pré ou peptide-signal, soit en plusieurs étapes lorsque cette séquence signal comprend, en plus de la séquence éliminée par la signal-peptidase, appelée séquence pré, une séquence éliminée plus tardivement au cours d'un ou plusieurs événements protéolytiques, appelée séquence pro.

L'invention concerne également les souches de levure, en particulier de Saccharomyces cerevisiae, transformées par le vecteur d'expression précédent, ainsi qu'un procédé de production d'une protéine d'intérêt qui comprend la culture de ces dernières en présence de galactose.

Elle concerne notamment les souches Saccharomyces cerevisiae transformées par le vecteur d'expression ci-dessus, qui ont été déposées à la Collection Nationale de Cultures de Microorganismes (C.N.C.M.) - Institut Pasteur - 28, rue du Docteur Roux,

### Paris - France :

- N ° l - 919 le 28 Décembre 1989
- N ° l - 1021 le 27 Décembre 1990
- N ° l - 1022 le 27 Décembre 1990
- N ° l - 1023 le 27 Décembre 1990

L'invention sera mieux comprise à l'aide des exemples ci-après:

Une grande partie de l'ensemble des techniques ci-après, bien connues de l'homme de l'art, est exposée en détail dans l'ouvrage de Maniatis et al.: "Molecular cloning : a Laboratory manual" publié en 1989 par les éditions Cold Spring Harbor Press à New York (2e édition).

La synthèse des oligonucléotides est réalisée à l'aide d'un synthétiseur d'ADN Biosearch 4600.

### EXEMPLE 1 : Détermination de la séquence de l'ADNc de l'urate oxydase d'A. flavus

### 1) Isolement des ARN messagers d'Aspergillus flavus

La souche d'A. flavus productrice d'urate oxydase a été cultivée dans des conditions de production d'urate oxydase, c'est-à-dire dans un milieu contenant de l'acide urique de composition suivante: glucose 15 g/I, MgS0₄,7H₂0 1 g/I, KH₂ PO₄ 0,75 g/I, CaC0₃ 1,2 g/I, acide urique 1,2 g/I, KOH 0,5 g/I, huile de soja 0,66 ml/l, FeS0₄,7H₂0 10 mg/l, CuS0₄,5H₂0 1 mg/1, ZnS0₄,7H₂0 3 mg/1, MnS0₄,H₂0 1 mg/I. Le milieu est ajusté à pH 7 avec H₂ S0₄ 1 M et est stérilisé à 120 ° C pendant 80 min.

Dans un erlenmeyer de 5 I on ensemence 1,5 I de milieu avec environ 1 à 3.10⁷ spores.

La culture est incubée pendant environ 40 h à 30 ° C sous agitation (120 tr/min). Le mycélium est récupéré par filtration sur gaze, lavé avec de l'eau et congelé dans l'azote liquide.

15 g de mycélium (poids humide) sont décongelés et resuspendus dans 45 ml de tampon de lyse puis repris dans un même volume de billes (0,45 µm de diamètre). Le tampon de lyse est constitué de thiocyanate de guanidine 4 M, Tris-HCI 10 mM pH 7,6, EDTA 10 mM, β-mercaptoéthanol 50 ml/I. La suspension mycélienne est broyée au broyeur Zellmühler (vibrogène) pendant 5 min.

Le broyat est récupéré et les billes décantées. Le surnageant est prélevé (environ 45 ml) et ramené à 3 M final en chlorure de lithium et conservé à 0 ° C.

Après deux jours, on le centrifuge pendant 60 min à 10 000 tr/min. Le surnageant est écarté et le culot est repris dans 40 ml de LiCI 3M et recentrifugé à 10 000 tr/min pendant 1 h 30.

On ajoute la protéinase K (SIGMA) 40 ug/ml du SDS (0,1 % P/V) et de l'EDTA à 20 mM. On incube à 37 ° C pendant 3 h. On précipite avec 2 volumes d'éthanol, puis on effectue un lavage à l'éthanol 70 %. On reprend le culot dans 0,5 ml de tampon TE (tris HCI 10 mM EDTA, 1 mM pH 7,5), on extrait 2 fois au chloroforme, on précipite à l'éthanol. Les ARN sont conservés à -80 _{°} C dans l'alcool.

### 2) Purification de la fraction poly A+ des ARN

Environ 1 mg d'ARN est précipité 20 min à 4 ° C (15 000 tr/min) puis lavé avec de l'éthanol 70 % puis séché. Le culot est repris dans 1 ml de tampon TE et resuspendu par agitation au vortex. L'oligo dT- cellulose type 3 (commercialisé par Collaborative Research Inc, Biomedicals Product Division) est préparé suivant les recommandations du fabricant. L'ARN est déposé sur l'oligo dT, agité doucement pour remettre en suspension les billes, puis chauffé pendant 1 min à 65 _{°} C.

La suspension est ajustée à 0,5 M NaCI puis mise à agiter doucement pendant 10 min. La suspension est alors centrifugée pendant 1 min à 1 000 tr/min, le surnageant est éliminé, le culot est lavé 2 fois avec 1 ml de tampon TE contenant 0,5 M NaCI. Les surnageants sont éliminés. L'élution de la fraction polyadénylée des ARN (constituée des ARN messagers) est obtenue par suspension des billes dans 1 ml de tampon TE, puis chauffage de cette suspension à 60 ° C pendant 1 min, suivie d'une agitation pendant 10 min sur plaque basculante. On centrifuge ensuite 1 min à 1 000 tr/min, ce qui permet de récupérer d'une part le surnageant contenant des ARNm libres en solution et d'autre part le culot de billes de cellulose. L'ensemble des opérations ci-dessus (à partir de l'élution) est répété. Les surnageants ainsi obtenus sont rassemblés, on élimine l'excés de billes par centrifugation et on précipite le surnageant à l'éthanol contenant du NaCI suivant les techniques habituelles. (Maniatis: op. prec.).

### 3) Constitution de la banque des ADNc

A partir des ARN messagers isolés comme décrit dans le paragraphe précédent, on a réalisé une banque d'ADNc dans le vecteur pTZ19R (commercialisé par PHARMACIA). Ce vecteur est un plasmide comprenant un polylinker contenant des sites uniques de restriction.

La technique de clonage utilisée est celle décrite par Caput et al, (technique de l'amorce-adapteur (Primer-adapter) : (Caput et al, Proc. Natl. Acad. Sci. (U.S.A.) (1986) 83, 1670-1674).

Elle consiste d'une part à digérer le vecteur par Pst1, ajouter une queue de polydC sur l'extrémité 3' protubérante, puis à digérer les plasmides ainsi obtenus par BamHl. Le fragment correspondant au vecteur est purifié sur colonne de Sépharose CL4B (Pharmacia). Il comprend donc une queue polydC a une extrémité, l'autre extrémité étant cohésive, du type BamHl. D'autre part, les ARN messagers sont soumis à la transcription inverse à partir d'une amorce dont la séquence est la suivante 5'>GATCCGGGCCCT₍₁₂₎>3. Ainsi, les ADNc présentent-ils à leur extrémité 5' la séquence GATCC complémentaire de l'extrémité cohésive BamHl.

Les hybrides ARN-ADN obtenus par action de la transcriptase inverse sont soumis à une hydrolyse alcaline qui permet de se débarrasser de l'ARN. Les ADNc simple brin sont alors purifiés par 2 cycles sur colonne de Sépharose CL4B et soumis à un traitement à la terminal transférase de façon à ajouter des polydG en 3'. Les ADNc sont insérés sous forme simple brin dans le vecteur préparé comme décrit plus haut. Un second oligonucléotide "l'adapter" complémentaire de l'amorce est nécessaire pour générer un site BamHl "ouvert" à l'extrémité 5' des ADNc. Après hybridation du vecteur, de l'ADNc et de 1"'adapter", les molécules recombinantes sont circularisées par l'action de la ligase du phage T4. Les régions simple brin sont alors réparées grâce à l'ADN polymérase du phage T4.

Le pool de plasmides ainsi obtenu sert à transformer la souche MC 1061 pour la résistance à l'ampicilline (Casabadan Chou et Cohen J. Bact. (1980) 143 pages 9971-980).

### 4) Détermination de la séquence partielle de l'urate oxydase

Une préparation d'urate oxydase d'A. flavus (SIGMA) a été repurifiée par chromatographie sur une colonne Red-agarose 120 (SIGMA), suivie d'une filtration sur un gel acrylamide-agarose Ultrogel Aca 44 (IBF).

Afin d'obtenir des informations sur la séquence des acides aminés de l'urate oxydase purifiée, permettant la synthèse des sondes nécessaires au clonage de l'ADNc, un séquençage amino-terminal direct de la protéine a été tenté. Ce dernier n'a pas abouti, probablement à cause d'un blocage amino-terminal de la protéine.

La stratégie ci-après a donc été développée pour l'obtention de la séquence partielle de l'urate oxydase
- coupure de la protéine par des enzymes protéolytiques (à l'aide des enzymes trypsine et protéase V8 de Staphylococcus aureus)
- séparation des polypeptides obtenus par HPLC phase inverse
- séquençage des peptides purifiés.

### 1) Hydrolyse de l'urate oxydase par la trypsine, purification et séquençage des peptides :

l'urate oxydase à 9 mg/ml dans un tampon de carbonate d'ammonium 100 mM pH 8,9 a été digéré par la trypsine (Worthington, TPCK) avec un rapport urate oxydase/trypsine de 30/1 en poids à 30 ° C pendant 24 h. Après l'hydrolyse tryptique, 60 µg d'urate oxydase digérée ont été directement injectés sur une colonne HPLC phase inverse de silice greffée Brownlee G18 (colonne 10 x 0,2 cm), équilibrée en acétonitrile 1 % (v/v), acide trifluoro-acétique 0,1 % (v/v) dans l'eau. Les peptides ont été ensuite élués par un gradient linéaire d'acétonitrile dans une solution d'acide trifluoroacétique (0,1 % v/v) dans l'eau variant de 1 % à 60 % d'acétonitrile en 60 min, avec un débit de 150 ul/min. Les peptides à la sortie de la colonne ont été détectés par mesure de la densité optique à 218 nm.

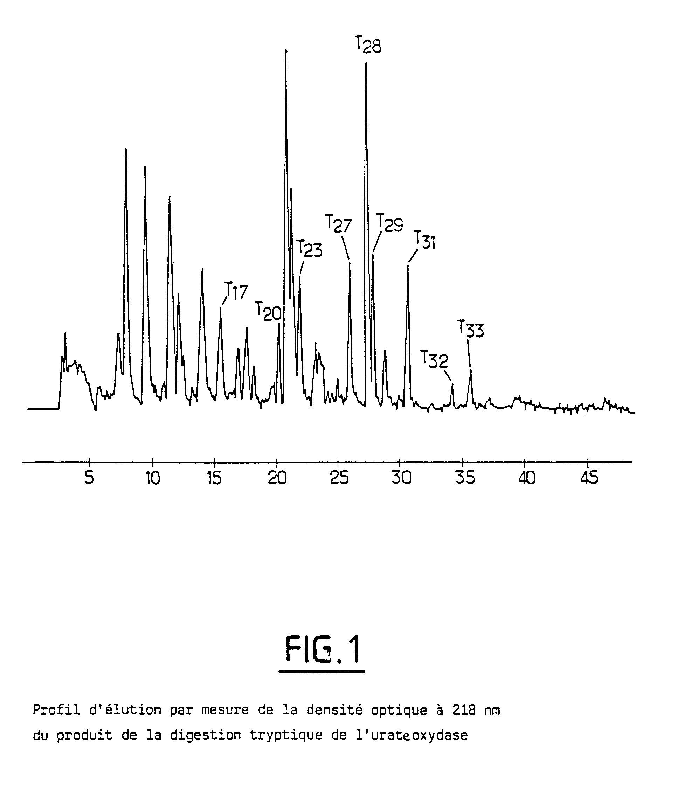

Le profil d'élution est présenté dans la figure 1, dans laquelle les numéros suivant la lettre T (Trypsine) correspondent aux pics identifiés.

Chaque pic a été collecté et conservé à -20 _{°} C jusqu'au moment de l'analyse sur un séquenceur de protéines (le modèle 470 A d'Applied Biosystems), équipé d'un chromatographe (modèle 430 A d'Applied Biosystems) qui analyse en continu les dérivés phényl-thiohydantoïques formés, après chaque cycle de dégradation.

Le tableau (1) ci-après présente les séquences peptidiques des 9 pics identifiés.

### 2) Hydrolyse de l'urate oxydase par la protéase V8, purification et séquençage des peptides :

L'urate oxydase, à une concentration de 2 mg/ml dans un tampon acétate d'ammonium 100 mM pH 6,8, a été digérée par la protéase V8 de Staphylococcus aureus (Boehringer-Mannheim) avec un rapport urate oxydase/protéase V8 de 60/1 à 30 ° C pendant 72 h. 160 µg d'urate oxydase digérée ont ensuite été injectés sur une colonne HPLC phase inverse de silice greffée Brownlee G18 (colonne 10 x 0,2 cm) ; particules (7 x 0,03 um) équilibrée en acétonitrile 1 %, acide trifluoroacétique 0,1 % (v/v) dans l'eau. Les peptides ont ensuite été élués par un gradient linéaire d'acétonitrile dans une solution d'acide trifluoroacétique dans l'eau (0,1 % (v/v)) variant de 1 % à 60 % d'acétonitrile en 60 min, avec un débit de 150 µl/ml. Les peptides à la sortie de la colonne ont été détectés par mesure de la densité optique à 218 nm.

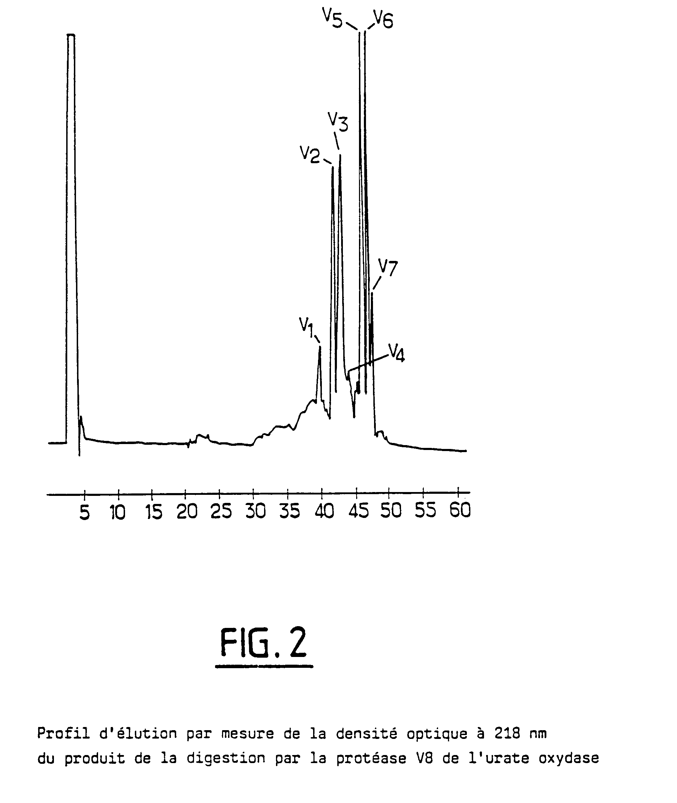

Le profil d'élution est présenté dans la figure 2, dans laquelle les numéros suivant la lettre V (protéase V8) correspondent aux pics identifiés.

Chaque pic a été collecté et conservé à -20 ° C jusqu'au moment de l'analyse sur le séquenceur de protéines déjà mentionné.

Le tableau (1) ci-après présente les séquences peptidiques des 5 pics identifiés.

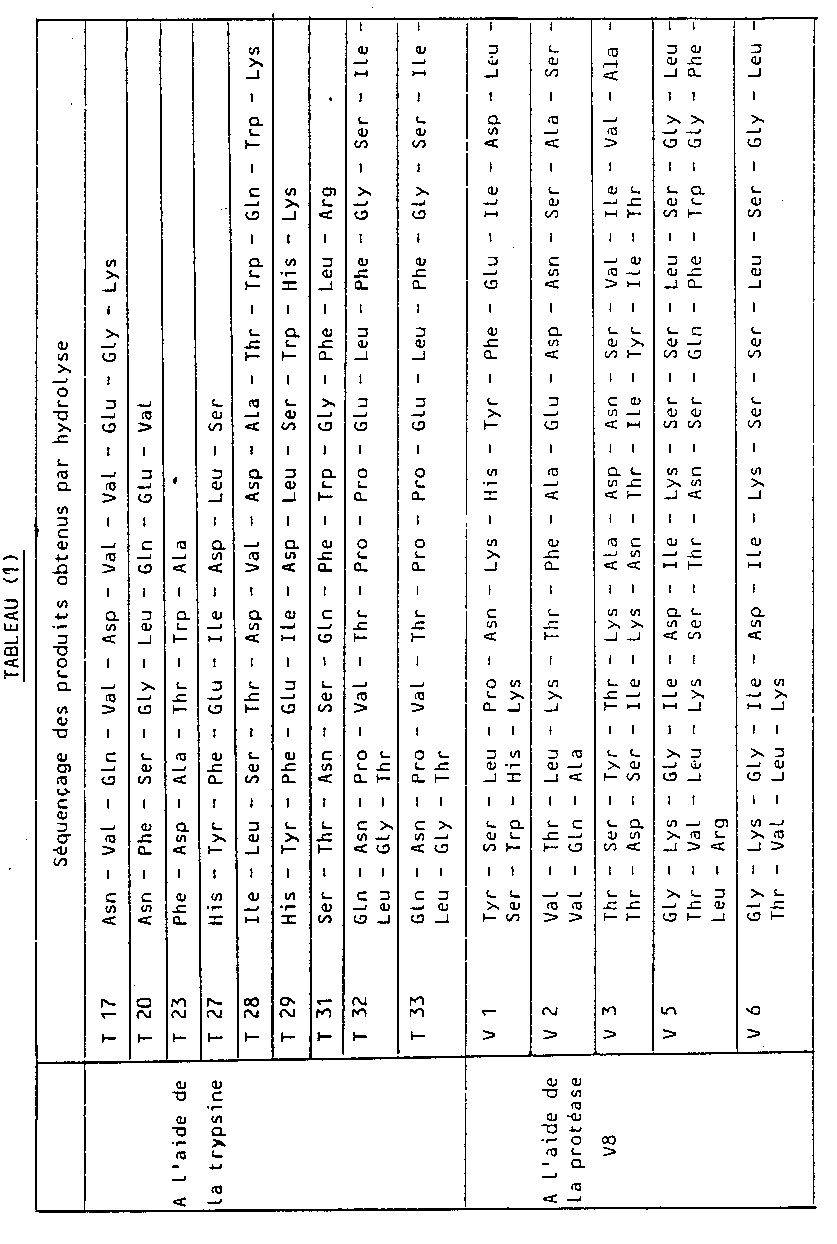

### 5) Criblage des bactéries

### 1) Préparation des sondes marquées :

Deux pools de sondes déduites de séquences en aminoacides de la protéine ont été synthétisés à l'aide d'un synthétiseur d'ADN Biosearch 4600. Le premier pool correspond à la séquence de résidus His-Tyr-Phe-Glu-lle-Asp (partie de la séquence de T 27), soit de 5' vers 3' : 
    A     T     G     G     G
T C G A T   T C   A A   T A   T G
    T     C     A     A     A
 Ce pool est en fait constitué de 2⁴ x 3 = 48 oligonucléotides différents représentant toutes les combinaisons possibles.

Le deuxième pool correspond à la séquence en résidus aminoacides Gln-Phe-Trp-Gly-Phe-Leu (partie de la séquence de V5), soit de 5' vers 3' : 
GG  A      G  T
A  AAGCCCCA AA TG
 AA  C      A  C
     T
 Ce pool est constitué de 2⁴ x 4 = 64 combinaisons.

Les sondes sont marquées à la terminale désoxynucléotide transférase (TdT) (commercialisé par IBI, Inc).

La réaction s'effectue sur 100 ng d'un mélange d'oligonucléotides en solution (100 mg/ml) dans du tampon de réaction "Cobalt" (fourni 10 fois concentrée par IBI inc. : 1,4 M de cocodylate de potassium-pH 7,2, 300 mM de dithiothréitol, 1 ul d'enzyme désoxynucléotidyl-terminal-transférase (IBI Inc) et 50 µCi de désoxycytidyltriphosphate dCTP marqué au P32.

La réaction se fait à 37 _{°} C pendant 10 min puis est arrêtée en ajoutant 1 ul d'EDTA 0,5 M.

On extrait au phénol et on dialyse sur colonne de polyacrylamide Biogel P 10 (Biorad: 150-1050).

### 2) Hybridation et détection des colonies contenant l'ADNc de l'urate oxydase :

Environ 40 000 colonies sont criblées par la technique d'hybridation in situ mise au point par Grunstein et Hogness (1975, Proc. Natl. Acad. Sci. (U.S.A.), 72 3961). Environ 6 000 bactéries sont étalées sur boites de Pétri de façon à obtenir des colonies isolées. Après incubation pendant 24 h à 37 ° , chaque boite est repliquée sur 2 filtres, chaque filtre étant destiné à être traité avec un des 2 pools de sondes, de façon que toutes les colonies obtenues soient testées avec les 2 pools de sondes en parallèle.

Les filtres sont mis à hybrider avec un des 2 pools de sondes dans un tampon contenant 6 x SSC, 10 x Denhardt's et 100 µg/ml d'ADN de sperme de saumon soniqué et dénaturé (SIGMA). La température d'hybridation est de 42 ° C et la durée de 16 h. La solution 6 x SSC s'obtient par dilution d'une solution 20 x SSC. La préparation du tampon 20 x SSC est décrite dans Maniatis, Fritsch et Sambrook (op. cité). En résumé, ce tampon contient 175,3 g/I de NaCI; 88,2 g/I de citrate de sodium et est ajusté à pH 7 par quelques gouttes de NaOH 10N. La solution 10 x Denhardt's contient 1 g de Ficoll, 1 g de polyvinylpyrroli- done, 1 g de sérum-albumine humaine pour 500 ml de volume final.

Après lavage dans la solution 6 x SSC à 42 ° C (3 h avec 5 changements de bain), les filtres sont essuyés au papier Joseph et mis en autoradiographie. Les filtres sont révélés après 16 h. Il est apparu qu'une fraction d'environ 0,5% des colonies hybridait avec les 2 pools de sondes.

5 colonies parmi celle-ci ont été reprises et purifiées. On a préparé l'ADN plasmidique de chacune de ces colonies, et l'on a analysé cet ADN par digestion avec soit BamHl, soit Hindlll, soit à la fois BamHl et Hindlll.

Après analyse sur gel d'agarose, il est apparu que les 5 plasmides obtenus sont linéarisés par BamHl et par Hindlll. Les doubles digestions permettent de libérer un fragment correspondant à l'intégralité de l'ADNc cloné. La taille de ce fragment est d'environ 1,2 Kb dans 3 cas, d'environ 0,9 Kb dans les 2 autres cas. Pour la détermination ci-après on a sélectionné un des fragments de 0,9 Kb et un des fragments de 1,2 Kb qui ont été reclonés (voir point 6 ci-aprés).

### 6) Détermination de la séquence de l'ADNc de l'urate oxydase

On a recloné un des fragments de 0,9 Kb d'une part (clone 9A) et un des fragments de 1,2 Kb (clone 9C) d'autre part, dans l'ADN de la forme réplicative de phage simple-brin M13. L'ADN des clones M13 contenant d'une part le fragment de 0,9 Kb et d'autre part le fragment de 1,2 Kb a été soumis à une digestion exonucléasique de manière à générer une série de clones M13 chevauchants (procédure "Cyclone 1 Biosystem" de IBI). Ces derniers ont été séquencés par la méthode des didéoxyribonucléotides (Sanger et al, PNAS-U.S.A.-1977, 14, 5463-5467).

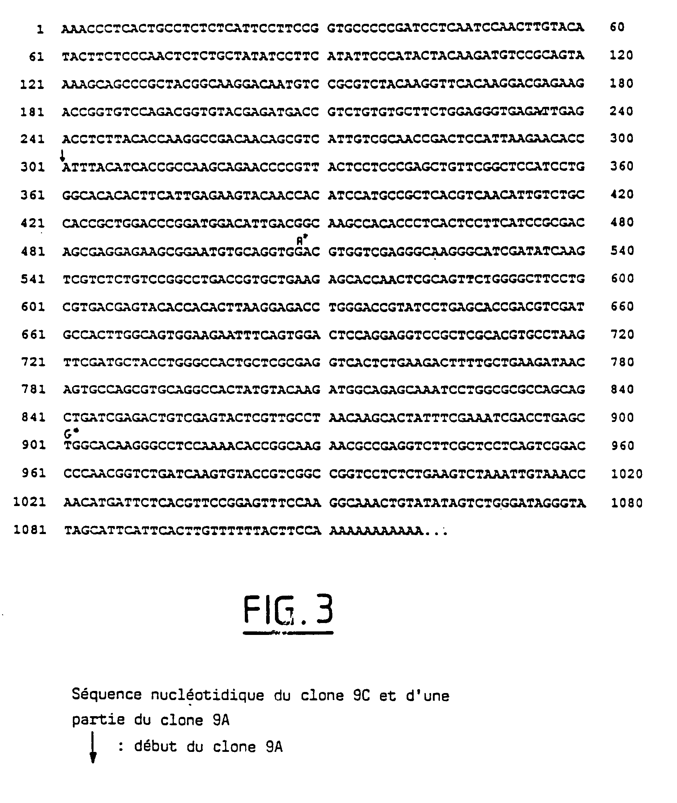

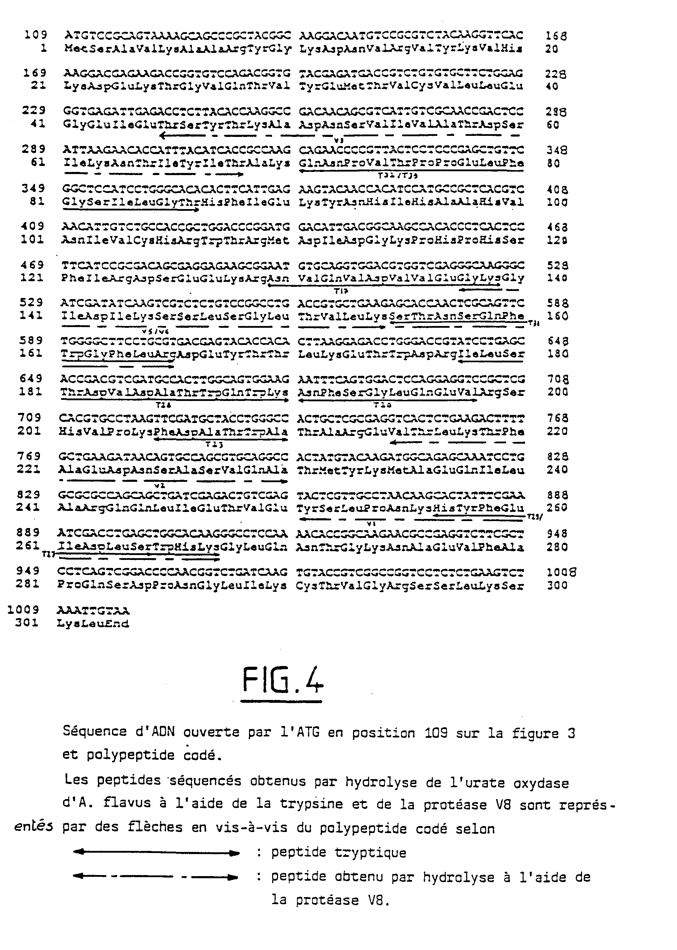

La séquence nucléotidique du clone 9C est représentée sur la figure 3 laquelle indique également par une flèche le début du clone 9A et par un symbole nucléotidique muni d'un astérisque les nucléotides séquencés du clone 9A non identiques à ceux du clone 9C (quand on fait correspondre les deux séquences et les sites de restriction Accl et BamHl utilisés dans les constructions ultérieures (Cf. 2).

On constate que :
- la séquence nucléotidique du fragment le plus long (clone 9C) recouvre celle du fragment le plus court (clone 9A), à deux différences près (v figure 3). Une des différences est silencieuse, l'autre correspond à un changement d'un résidu tryptophane en résidu glycine. Ces différences peuvent être dues, soit à des différences dans les ARN messagers isolés, (Cf. 2) ci-dessus soit à des erreurs de la transcriptase inverse utilisée lors de la constitution de la banque des ADNc (Cf. 3) ci-dessus.

Dans le cas du fragment le plus long, un codon ATG (en position 109 sur la figure 3) ouvre une phase ouverte correspondant à un polypeptide de 302 aminoacides, de masse moléculaire voisine de 34240 Da, dont la séquence correspond à la séquence partielle de l'urate oxydase d'A. flavus purifiée (Cf. 4).

On a représenté sur la figure 4 la séquence d'ADN ouverte par le codon ATG et le polypeptide codé, ainsi que par des flèches en vis-à-vis avec le polypeptide codé les peptides séquencés (Cf. 4)) obtenus par hydrolyse de l'urate oxydase d'A. flavus à l'aide de la trypsine et de la protéase V8.

On constate que la séquence du polypeptide se termine par le triplet Ser-Lys-Leu, typique des enzymes à localisation peroxisomale (Gould S.J. et al, J. Cell, Biology 108 (1989) 1657-1664).

### EXEMPLE 2 : Construction de trois vecteurs d'expression de l'ADNc de l'urate oxydase dans la levure, le plasmide pEMR469 porteur d'un promoteur ADH₂ et le plasmide pEMR473 ainsi que le plasmide pEMR515 porteurs du promoteur artificiel de l'invention

La stratégie mise en oeuvre fait appel à des fragments obtenus à partir de plasmides préexistants accessibles au public et à des fragments préparés par voie de synthèse selon les techniques maintenant couramment utilisées. Les techniques de clonage employées sont celles décrites par T. MANIATIS, EF. FRITSCH et J. SAMBROOK dans "Molecular Cloning, a laboratory manual" (Cold Spring Harbor Laboratory, 1984). La synthèse des oligonucléotides est réalisée à l'aide d'un synthétiseur d'ADN Biosearch 4 600.

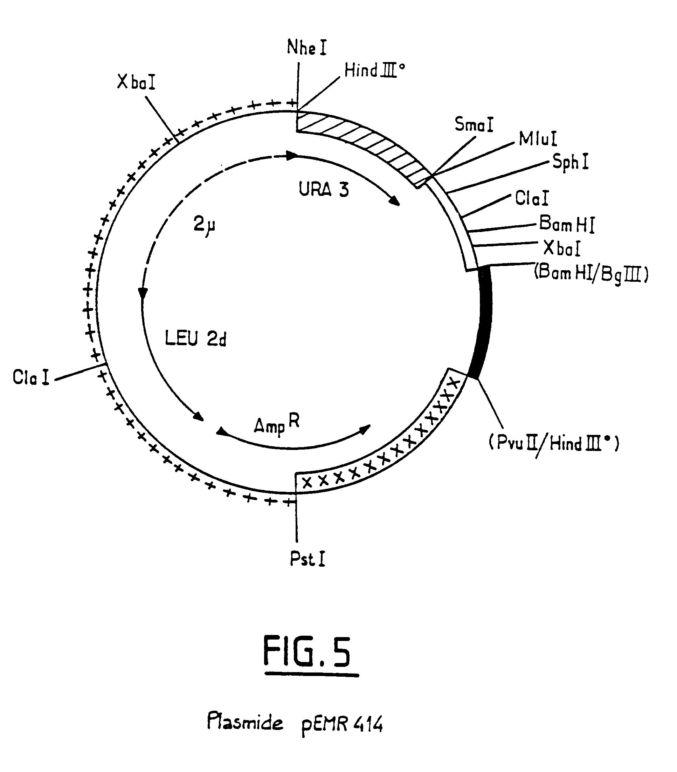

La description ci-après sera mieux comprise à la lecture des figures 5, 6 et 7 qui représentent respectivement des cartes de restriction du plasmide pEMR414, et des plasmides pEMR469 ainsi que pEMR473. Les symboles utilisés sur ces figures seront précisés dans l'exposé ci-après. Dans le cas où un site a été rendu franc par la polymérase de Kleenow, il lui est affecté l'indice " _{°} "; lorsque les sites ont été supprimés par ligation, ils sont indiqués entre parenthèses.

### 1) Construction du plasmide pEMR469 :

Ce plasmide a été construit à partir du vecteur navette E. coli-levure pEMR414, construit par ligations successives des éléments ci-après :
- le fragment Pstl-Hindlll _{°} - symbolisé par ++++ sur la figure 5 - du plasmide pJDB207 (BEGGS, 1978 : Gene cloning in yeast-p. 175-203 dans : Genetic Engineering vol 2 - WILLIAMSON - Academic Press - London UK) comprenant la partie amont du gène de résistance à l'ampicilline Amp^{R} de pBR322 (Sutcliffe 1979 Cold Spring Symp. Quart. Biol. 43, 779) et un fragment de 2 u endogène forme B portant le gène LEU2 de S. cerevisiae partiellement délété de son promoteur (appelé LEU2d), le locus STB (REP3) et l'origine de réplication du 2 u (HARTLEY et DONELSON, 1980, Nature, 286, 860-865). L'extrémité Hindlll de ce fragment a été rendue franche par action de la polymérase de Kleenow. Elle est notée Hindlll _{°} sur la figure 5.
- le fragment Hindlll-Smal - représenté par 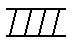 sur la figure 5 - du chromosome V de levure contenant le gène URA3 avec son promoteur (ROSE et al., 1984, Gene, 29, p. 113-124). Ce fragment Hindlll-Smal provient du plasmide pFL1 (CHEVALLIER et al., 1980, Gene 11, 11-19)). L'extrémité Hindlll de ce plasmide a été rendue franche par l'action de la polymérase de Kleenow.
- un fragment Saml-BamHl - symbolisé par 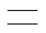 sur la figure 5 - contenant une version synthétique du promoteur du gène ADH2 ne différant de la version naturelle décrite par RUSSEL et SMITH (RUSSEL et al., 1983). J. Biol. Chem. 258, 2674-2682) que par quelques paires de bases destinées à introduire des sites de restriction. (La séquence naturelle pourrait être utilisée avec des résultats peu différents).

La séquence de ce fragment est donnée ci-après : 
S M
m l
a u
I I
GGGACGCGTCTCCTCTGCCGGAACACCGGGCATCTCCAACTTATAAGTTGGAG
CCCTGCGCAGAGGAGACGGCCTTGTGGCCCGTAGAGGTTGAATATTCAACCTC

AAATAAGAGAATTTCAGATTGAGAGAATGAAAAAAAAAAAAAAAAAAAAGGCAGAGGAGA
TTTATTCTCTTAAAGTCTAACTCTCTTACTTTTTTTTTTTTTTTTTTTTCCGTCTCCTCT

S
p
h
I
GCATAGAAATGGGGTTCACTTTTTGGTAAAGCTATAGCATGCCTATCACATATAAATAGA
CGTATCTTTACCCCAAGTGAAAAACCATTTCGATATCGTACGGATAGTGTATATTTATCT

GTGCCAGTAGCGACTTTTTTCACACTCGAGATACTCTTACTACTGCTCTCTTGTTGTTTT
CACGGTCATCGCTGAAAAAAGTGTGAGCTCTATGAGAATGATGACGAGAGAACAACAAAA

TATCACTTCTTGTTTCTTCTTGGTAAATAGAATATCAAGCTACAAAAAGCATACAATCAA
ATAGTGAAGAACAAAGAAGAACCATTTATCTTATAGTTCGATGTTTTTCGTATGTTAGTT

B
C a
l m
a H
I I
CTATCAACTATTAACTATATCGATACCATATGGATCCGTCGACTCTAGAGGATCGTC
GATAGTTGATAATTGATATAGCTATGGTATACCTAGGCAGCTGAGATCTCCTAGCAG

B
a
m
H
I
GACTCTAGAG
CTGAGATCTCCTAG
- le fragment Bglll-Hindlll - symbolisé par - sur la figure 5 - portant l'extrémité 3' du gène PGK de levure. Ce fragment provient de la digestion complète à l'aide de Bglll du fragment Hindlll de l'ADN chromosomique de levure, portant le gène PGK décrit par HITZEMANN et al. (1982 Nucleic Acids Res., 10, 7791-7808) lequel ne présente qu'un site Bglll. Cette digestion permet d'obtenir deux fragments Hindlll-Bglll, dont le plus petit d'environ 0,4 Kb, qui porte l'extrémité 3' du gène PGK de levure est retenu. La séquence de ce dernier fragment est décrite par HITZEMANN et al. (référence citée ci-dessus). Le site Bglll est cloné dans le site BamHl du fragment précédent (les sites BamHl et Bglll disparaissent donc) et le site Hindlll, rendu franc par action de la polymérase de Kleenow, est cloné dans le site Pvull du fragment de Pvull-Pstl pBR322 décrit ci-après.
- le fragment Pvull-Pstl - symbolisé par 77 sur la figure 5 - de pBR322 contenant l'origine de réplication et la partie aval du gène de résistance à l'ampicilline Amp^{R}.

Le plasmide pEMR414 ainsi constitué comporte donc les éléments suivants :
- une origine de réplication et un gène de résistance à l'ampicilline Amp^{R} permettant la réplication et la sélection du plasmide dans les cellules E. coli. Ces éléments permettent la transformation dans les cellules de E. coli.
- une origine de réplication pour la levure (ARS), le locus STB et le gène LEU2 de S. cerevisiae sans promoteur et le gène URA3 avec son promoteur de S. cerevisiae. Ces éléments permettent la réplication et la sélection du plasmide dans les cellules de S. cerevisiae ainsi qu'une efficacité de partition suffisante dans les cellules contenant le plasmide 2µ, endogène.

Le plasmide pEMR414 a été soumis à une digestion complète par les enzymes de restriction Nhel et Clal. Le petit fragment Nhel-Clal contenant le gène URA3, appelé ci-après fragment A, a été purifié.

Le plasmide pEMR414 a été soumis à une digestion complète par les enzymes Nhel et BamHl. Le grand fragment Nhel-BamHl contenant notamment le gène LEU2d et l'origine de réplication du plasmide pBR322, appelé ci-après fragment B, a été purifié.

On a préparé d'autre part le fragment synthétique Clal-Accl contenant le début d'un gène codant pour la protéine déduite de la séquence de l'ADNc de l'urate oxydase (clone 9C). Ce fragment comporte des modifications par rapport au clone 9C, introduites dans le but de mettre en place des codons usuels dans la levure (Cf. SHARP et al., 1986, Nucl. Ac. Res. Vol. 14, 13, pp. 5125-5143) sans changement des acides aminés codés. La séquence de ce fragment, dénommé ci-après fragment C, est la suivante (les nucléotides soulignés sont ceux modifiés par rapport au clone 9C).

C                                                    A
l                                                    c
a                                                    c
I                                                    I

CGATATACACAATGTCTGCTGTTAAGGCTGCTAGATACGGTAAGGACAACGTTAGAGT
---+---------+---------+---------+---------+---------+----
  TATATGTGTTACAGACGACAATTCCGACGATCTATGCCATTCCTGTTGCAATCTCAGA

On a soumis le plasmide du clone 9C (Cf. figure 3) à une digestion par les enzymes Accl et BamHl. Le fragment Accl-BamHl qui contient la fin de l'ADNc de l'urate oxydase, ci-après appelé fragment D, a été purifié. Ce fragment a la séquence suivante :

AccI
CTACAAGGTTCACAAGGACGAGAAG
- --+---------+---------+
TGTTCCAAGTGTTCCTGCTCTTC
ACCGGTGTCCAGACGGTGTACGAGATGACC GTCTGTGTGCTTCTGGAGGGTGAGATTGAG
---------+---------+---------+ ---------+---------+---------+
TGGCCACAGGTCTGCCACATGCTCTACTGG CAGACACACGAAGACCTCCCACTCTAACTC
ACCTCTTACACCAAGGCCGACAACAGCGTC ATTGTCGCAACCGACTCCATTAAGAACACC
---------+---------+---------+ ---------+---------+---------+
TGGAGAATGTGGTTCCGGCTGTTGTCGCAG TAACAGCGTTGGCTGAGGTAATTCTTGTGG
ATTTACATCACCGCCAAGCAGAACCCCGTT ACTCCTCCCGAGCTGTTCGGCTCCATCCTG
---------+---------+---------+ ---------+---------+---------+
TAAATGTAGTGGCGGTTCGTCTTGGGGCAA TGAGGAGGGCTCGACAAGCCGAGGTAGGAC
GGCACACACTTCATTGAGAAGTACAACCAC ATCCATGCCGCTCACGTCAACATTGTCTGC
---------+---------+---------+ ---------+---------+---------+
CCGTGTGTGAAGTAACTCTTCATGTTGGTG TAGGTACGGCGAGTGCAGTTGTAACAGACG
CACCGCTGGACCCGGATGGACATTGACGGC AAGCCACACCCTCACTCCTTCATCCGCGAC
---------+---------+---------+ ---------+---------+---------+
GTGGCGACCTGGGCCTACCTGTAACTGCCG TTCGGTGTGGGAGTGAGGAAGTAGGCGCTG
AGCGAGGAGAAGCGGAATGTGCAGGTGGAC GTGGTCGAGGGCAAGGGCATCGATATCAAG
---------+---------+---------+ ---------+---------+---------+
TCGCTCCTCTTCGCCTTACACGTCCACCTG CACCAGCTCCCGTTCCCGTAGCTATAGTTC
TCGTCTCTGTCCGGCCTGACCGTGCTGAAG AGCACCAACTCGCAGTTCTGGGGCTTCCTG
---------+---------+---------+ ---------+---------+---------+
AGCAGAGACAGGCCGGACTGGCACGACTTC TCGTGGTTGAGCGTCAAGACCCCGAAGGAC
CGTGACGAGTACACCACACTTAAGGAGACC TGGGACCGTATCCTGAGCACCGACGTCGAT
---------+---------+---------+ ---------+---------+---------+
GCACTGCTCATGTGGTGTGAATTCCTCTGG ACCCTGGCATAGGACTCGTGGCTGCAGCTA
GCCACTTGGCAGTGGAAGAATTTCAGTGGA CTCCAGGAGGTCCGCTCGCACGTGCCTAAG
---------+---------+---------+ ---------+---------+---------+
CGGTGAACCGTCACCTTCTTAAAGTCACCT GAGGTCCTCCAGGCGAGCGTGCACGGATTC
TTCGATGCTACCTGGGCCACTGCTCGCGAG GTCACTCTGAAGACTTTTGCTGAAGATAAC
---------+---------+---------+ ---------+---------+---------+
AAGCTACGATGGACCCGGTGACGAGCGCTC CAGTGAGACTTCTGAAAACGACTTCTATTG
AGTGCCAGCGTGCAGGCCACTATGTACAAG ATGGCAGAGCAAATCCTGGCGCGCCAGCAG
---------+---------+---------+ ---------+---------+---------+
TCACGGTCGCACGTCCGGTGATACATGTTC TACCGTCTCGTTTAGGACCGCGCGGTCGTC
CTGATCGAGACTGTCGAGTACTCGTTGCCT AACAAGCACTATTTCGAAATCGACCTGAGC
---------+---------+---------+ ---------+---------+---------+
GACTAGCTCTGACAGCTCATGAGCAACGGA TTGTTCGTGATAAAGCTTTAGCTGGACTCG
TGGCACAAGGGCCTCCAAAACACCGGCAAG AACGCCGAGGTCTTCGCTCCTCAGTCGGAC
---------+---------+---------+ ---------+---------+---------+
ACCGTGTTCCCGGAGGTTTTGTGGCCGTTC TTGCGGCTCCAGAAGCGAGGAGTCAGCCTG
CCCAACGGTCTGATCAAGTGTACCGTCGGC CGGTCCTCTCTGAAGTCTAAATTGTAAACC
---------+---------+---------+ ---------+---------+---------+
GGGTTGCCAGACTAGTTCACATGGCAGCCG GCCAGGAGAGACTTCAGATTTAACATTTGG
AACATGATTCTCACGTTCCGGAGTTTCCAA GGCAAACTGTATATAGTCTGGGATAGGGTA
---------+---------+---------+ ---------+---------+---------+
TTGTACTAAGAGTGCAAGGCCTCAAAGGTT CCGTTTGACATATATCAGACCCTATCCCAT
TAGCATTCATTCACTTGTTTTTACTTCCA AAAAAAAAAAAAAAAAAAAAAAAAAAAAAA
---------+---------+---------+ ---------+---------+---------+
ATCGTAAGTAAGTGAACAAAAAATGAAGGT TTTTTTTTTTTTTTTTTTTTTTTTTTTTTT
AAAAAAAAAAAAAAAAAAAAAAGGGCCCG BamHI
---------+---------+--------
TTTTTTTTTTTTTTTTTTTTTCCCGGGCCT AG

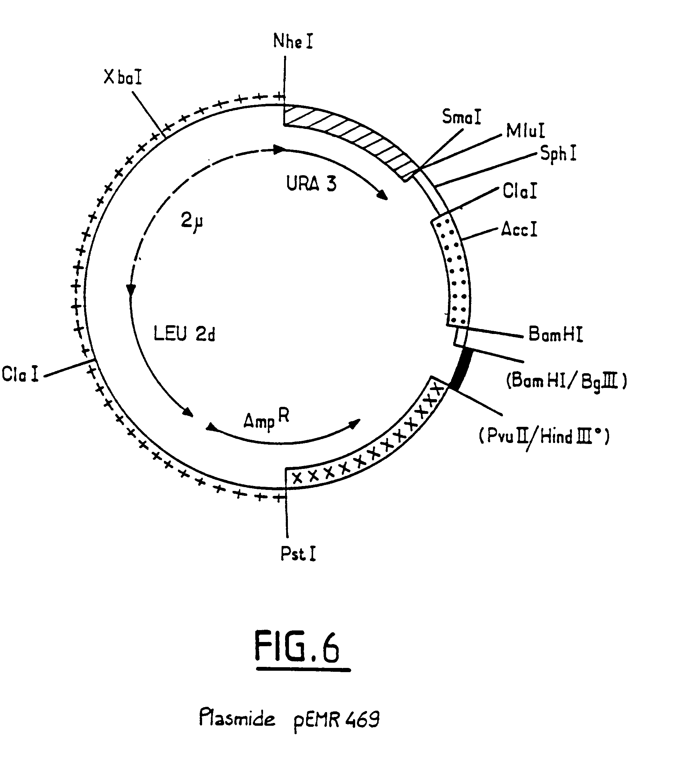

Les fragments A, B, C et D ont été ligués de manière à obtenir le plasmide pEMR469 représenté sur la figure 6, dans laquelle les symboles ont la même signification que sur la figure 5, les nouveaux fragments Clal-Accl et Accl-BamHl étant symbolisés par 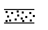

Le plasmide pEMR469 est porteur d'une séquence codant pour la protéine déduite de la séquence de l'ADNc de l'urate oxydase, sous le contrôle d'un promoteur appelé promoteur ADH₂, proche du promoteur ADH₂ naturel, comprenant la séquence :

M
l
u
I
CGCGTCTCCTCTGCCGGAACACCGGGCATCTCCAACTTATAAGTTGGAG
--+---------+---------+---------+---------+---------+----
AGAGGAGACGGCCTTGTGGCCCGTAGAGGTTGAATATTCAACCTC

AAATAAGAGAATTTCAGATTGAGAGAATGAAAAAAAAAAAAAAAAAAAAGGCAGAGGAGA
---+---------+---------+---------+---------+---------+------
TTTATTCTCTTAAAGTCTAACTCTCTTACTTTTTTTTTTTTTTTTTTTTCCGTCTCCTCT

S
p
h
I
Elément TATA
GCATAGAAATGGGGTTCACTTTTTGGTAAAGCTATAGCATGCCTATCACATATAAATAGA
---+---------+---------+---------+---------+---------+------
CGTATCTTTACCCCAAGTGAAAAACCATTTCGATATCGTACGGATAGTGTATATTTATCT

XhoI
GTGCCAGTAGCGACTTTTTTCACACTCGAGATACTCTTACTACTGCTCTCTTGTTGTTTT
---+---------+---------+---------+---------+---------+------
CACGGTCATCGCTGAAAAAAGTGTGAGCTCTATGAGAATGATGACGAGAGAACAACAAAA

TATCACTTCTTGTTTCTTCTTGGTAAATAGAATATCAAGCTACAAAAAGCATACAATCAA
---+---------+---------+---------+---------+---------+------
ATAGTGAAGAACAAAGAAGAACCATTTATCTTATAGTTCGATGTTTTTCGTATGTTAGTT
Région d'initiation de
la transcription

C
l
a
I
CTATCAACTATTAACTATAT
---+---------+-------
GATAGTTGATAATTGATATAGC

### 2) Construction du plasmide pEMR473 :

Le plasmide pEMR469 a été soumis à une digestion complète par les enzymes Mlul et Sphl. Le grand fragment Mlul-Sphl contenant le gène de l'urate oxydase a ensuite été ligué au fragment synthétique, de séquence donnée ci-après, correspondant à une partie (200 pb) de la séquence en amont de l'élément TATA du promoteur GAL7 de S. cerevisiae, laquelle comprend deux séquences d'activation amont de haute affinité appelées UAS1 et UAS2, encadrées ci-dessous (Cf. R. J. BRAM et al. (1986) EMBO J. vol. 5 n ° 3, p. 603-608).

M
l
u
I
UAS1
CGCGTCTATACTTCGGAGCACTGTTGAGCGAAGGCTCATTAGATATATTTTCTGTCAT
--♦-------♦---------♦---------♦---------♦---------♦-----
AGATATGAAGCCTCGTGACAACTCGCTTCCGAGTAATCTATATAAAAGACAGTA
UAS2
TTTCCTTAACCCAAAAATAAGGGAGAGGGTCCAAAAAGCGCTCGGACAACTGTTGACCGT
----♦---------♦---------♦---------♦---------♦---------♦-----
AAAGGAATTGGGTTTTTATTCCCTCTCCCAGGTTTTTCGCGAGCCTGTTGACAACTGGCA

GATCCGAAGGACTGGCTATACAGTGTTCACAAAATAGCCAAGCTGAAAATAATGTGTAGC
----♦---------♦---------♦---------♦---------♦---------♦-----
CTAGGCTTCCTGACCGATATGTCACAAGTGTTTTATCGGTTCGACTTTTATTACACATCG

S
p
h
I
CTTTAGCTATGTTCAGTTAGTTTGGCATG
----♦---------♦---------♦-----
GAAATCGATACAAGTCAATCAAACC

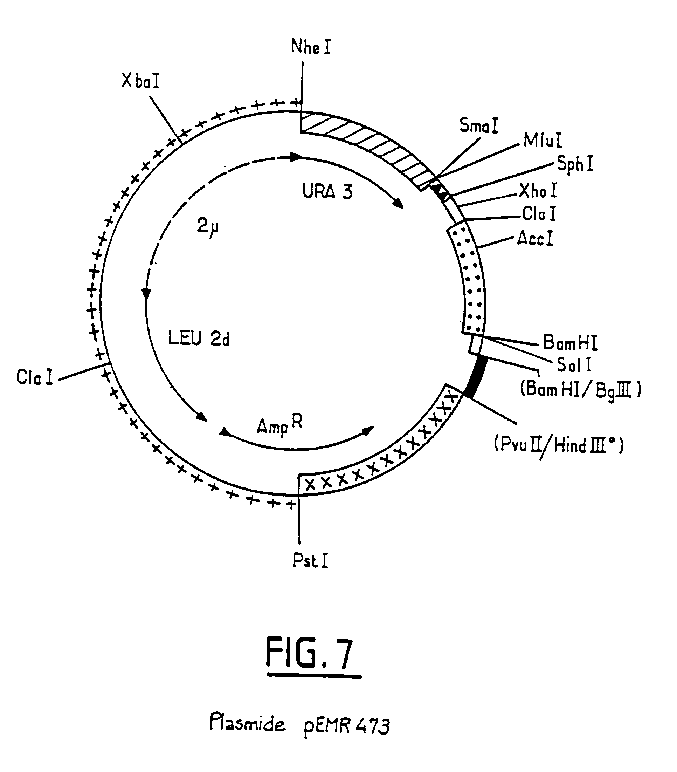

Le plasmide pEMR473 ainsi obtenu est représenté sur la figure 7, dans laquelle les symboles ont la même signification que dans la figure 6, le nouveau fragment Mlul-Sphl introduit étant symbolisé par 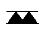

Le plasmide pEMR473 est donc porteur d'une séquence codant pour la protéine déduite de la séquence de l'ADNc de l'urate oxydase, sous le contrôle du promoteur artificiel de l'invention, lequel comprend la séquence :

M
l
u
l
UAS1
CGCGTCTATACTTCGGAGCACTGTTGAGCGAAGGCTCATTAGATATATTTTCTGTCAT
AGATATGAAGCCTCGTGACAACTCGCTTCCGAGTAATCTATATAAAAGACAGTA
UAS2
TTTCCTTAACCCAAAAATAAGGGAGAGGGTCCAAAAAGCGCTCGGACAACTGTTGACCGT
AAAGGAATTGGGTTTTTATTCCCTCTCCCAGGTTTTTCGCGAGCCTGTTGACAACTGGCA
GATCCGAAGGACTGGCTATACAGTGTTCACAAAATAGCCAAGCTGAAAATAATGTGTAGC
CTAGGCTTCCTGACCGATATGTCACAAGTGTTTTATCGGTTCGACTTTTATTACACATCG
S
p
h
I
Elément TATA
CTTTAGCTATGTTCAGTTAGTTTGGCATGCCTATCACATATAAATAGA
GAAATCGATACAAGTCAATCAAACCGTACGGATAGTGTATATTTATCT
GTGCCAGTAGCGACTTTTTTCACACTCGAGATACTCTTACTACTGCTCTCTTGTTGTTTT
CACGGTCATCGCTGAAAAAAGTGTGAGCTCTATGAGAATGATGACGAGAGAACAACAAAA
TATCACTTCTTGTTTCTTCTTGGTAAATAGAATATCAAGCTACAAAAAGCATACAATCAA
ATAGTGAAGAACAAAGAAGAACCATTTATCTTATAGTTCGATGTTTTTCGTATGTTAGTT
C
l
a
I
Région d'initiation
de la transcription
CTATCAACTATTAACTATAT
GATAGTTGATAATTGATATAGC

### 3) Construction du plasmide pEMR515 :

Le plasmide pEMR473 a été soumis à une digestion partielle par l'enzyme Xbal et à une digestion totale par l'enzyme Mlul. Le grand fragment Xbal-Mlul a été purifié. Ce fragment contient notamment les séquences de l'origine de réplication et le locus STB du 2µ, le gène LEU2d, le gène de résistance à l'ampicilline Amp^{R}, l'origine de réplication du pBR322 et la cassette d'expression de l'urate oxydase. Il ne contient en revanche ni le gène URA3, ni la partie du 2µ, comprise entre les sites Xbal et Nhel.

Le grand fragment Xbal-Mlul a été recircularisé via l'adaptateur de séquence suivant comportant des extrémités cohésives Xbal modifié et Mlul :
Xbal modifié

CTAGGCTAGCGGGCCCGCATGCA
CGATCGCCCGGGCGTACGTGCGC
MluI

Le plasmide pEMR515 ainsi obtenu ne possède qu'un seul des trois éléments du site FRT cible de la recombinase codée par le gène FLP du 2u.

Le plasmide pEMR515 est donc porteur d'une séquence codant pour la protéine déduite de la séquence de l'ADNc de l'urate oxydase sous le contrôle du promoteur artificiel de l'invention.

### EXEMPLE 3 : Transformation de la souche de levure EMY761, par les plasmides pEMR469, pEMR473 et pEMR515 - Transformation des souches de levures EMY500 et GRF18 par le plasmide pEMR515 - Transformation avec sélection pour la prototrophie de leucine

On a utilisé comme souches réceptrices trois souches de Saccharomyces cerevisiae non isogéniques :
- la souche EMY761 (Mata, leu2, ura3, his3)
- la souche EMY500 (Mata, leu2, ura3, pep4)
- la souche GRF18 (Mata, leu2, his3);

La souche GRF18 est bien connue de l'homme de l'art (Gerry FINK, MIT, USA). Les souches EMY761 et EMY500 sont apparentées à la souche GRF18. Elles ont été obtenues par croisements successifs de la souche GRF18 avec une souche ura3 dérivée de la souche FL100 (déposée à l'ATCC sous le n ° 28 383) et avec la souche 20B12 (Mata, tsp1, pep4) décrite par E.W. JONES (E.W. JONES et al. (1977) Genetics, 85, 23).

On peut obtenir la souche GRF18 par curage du plasmide pEMR515 de la souche GRF18 pEMR515 (leu+) déposée à la CNCM sous la référence n ° l-920, le 28 décembre 1989 et la souche EMY500 par curage du plasmide pEMR515 de la souche EMY500 pEMR515 (leu+) déposée à la CNCM sous la référence n ° l-919, le 28 décembre 1989.

Ces souches contiennent des mutations (leu2 et ura3), susceptibles d'être complémentées par le marqueur de sélection défectif LEU2d et le marqueur de sélection URA3, présents dans chacun des plasmides pEMR469 et pEMR473.

La technique de transformation utilisée est une variante de celle décrite par Beggs et al. (Beggs et al. (1978), Nature 275, 104-109). Elle consiste à soumettre les levures à un traitement de protoplastisation en présence d'un stabilisant osmotique, le sorbitol en concentration 1 M.

Le protocole précis de transformation est précisé ci-après :
a) 200 ml du milieu YPG liquide (Cf. tableau I) sont inoculés avec environ 5 x 10⁶ cellules d'une culture en phase stationnaire et la culture ainsi inoculée est placée une nuit sous agitation à 30 _{°} C.
b) Lorsque la culture atteint environ 10⁷ cellules par ml, les cellules sont centrifugées à 4 000 tr/min pendant 5 min et le culot est lavé avec du sorbitol 1 M.
c) Les cellules sont suspendues dans 5 ml de solution de sorbitol 1 M contenant 25 mM EDTA et 50 mM de dithiothréitol et incubées pendant 10 min à 30 ° C.
d) Les cellules sont lavées une fois avec 10 ml de sorbitol 1 M et suspendues dans 20 ml de sorbitol. De la zymolase-100T (préparation obtenue par purification partielle sur colonne d'affinité du surnageant de culture d'Arthobacter luteus et contenant de la β-1,3-glucane-laminaripentahydrolase, commercialisée par SEYKAGAKU KOGYO Co. Ltd) est ajoutée à une concentration finale de 20 µg/ml et on incube la suspension à température ambiante pendant environ 15 min.
e) Les cellules sont resuspendues dans 20 ml d'un milieu contenant du sorbitol appelé milieu YPG sorbitol (Cf. tableau 1 ci-après) et incubées pendant 20 min à 30 _{°} C sous agitation douce.
f) On centrifuge pendant 3 min à 2 500 tr/min.
g) On resuspend dans 9 ml de tampon de transformation (sorbitol 1 M, tris-HCI de pH 7,5 10 mM et CaC1₂ 10 mM).
h) On ajoute 0,1 ml de cellules et 5 µl de solution d'ADN (environ 5 µg) et on laisse la suspension obtenue pendant 10 à 15 min à température ambiante.

i) On ajoute 1 ml de la solution : polyéthylène glycol PEG 4000 20 %, tris-HCI de pH 7,5 10 mM et CaC1₂ 10 mM.
j) On verse 0,1 ml de la suspension obtenue en i) dans un tube contenant du milieu solide de régénération sans leucine (Cf. tableau 1 ci-après) préalablement fondu et maintenu liquide à environ 45 ° C. On verse la suspension sur une boite de Pétri contenant une couche solidifiée de 15 ml de milieu de régénération solide sans leucine.
k) On répète l'étape j) avec le reste de la suspension cellulaire obtenue en h).

Les transformés commencent à apparaître au bout de trois jours.

On a ainsi retenu un transformé EMY761 pEMR469 (leu+), et trois transformés EMY761 pEMR473 (leu+) (clones 1, 2 et 3), un transformé EMY761 pEMR515 (leu+), un transformé EMY500 pEMR515 (leu+) et un transformé GRF18 pEMR515 (leu+).

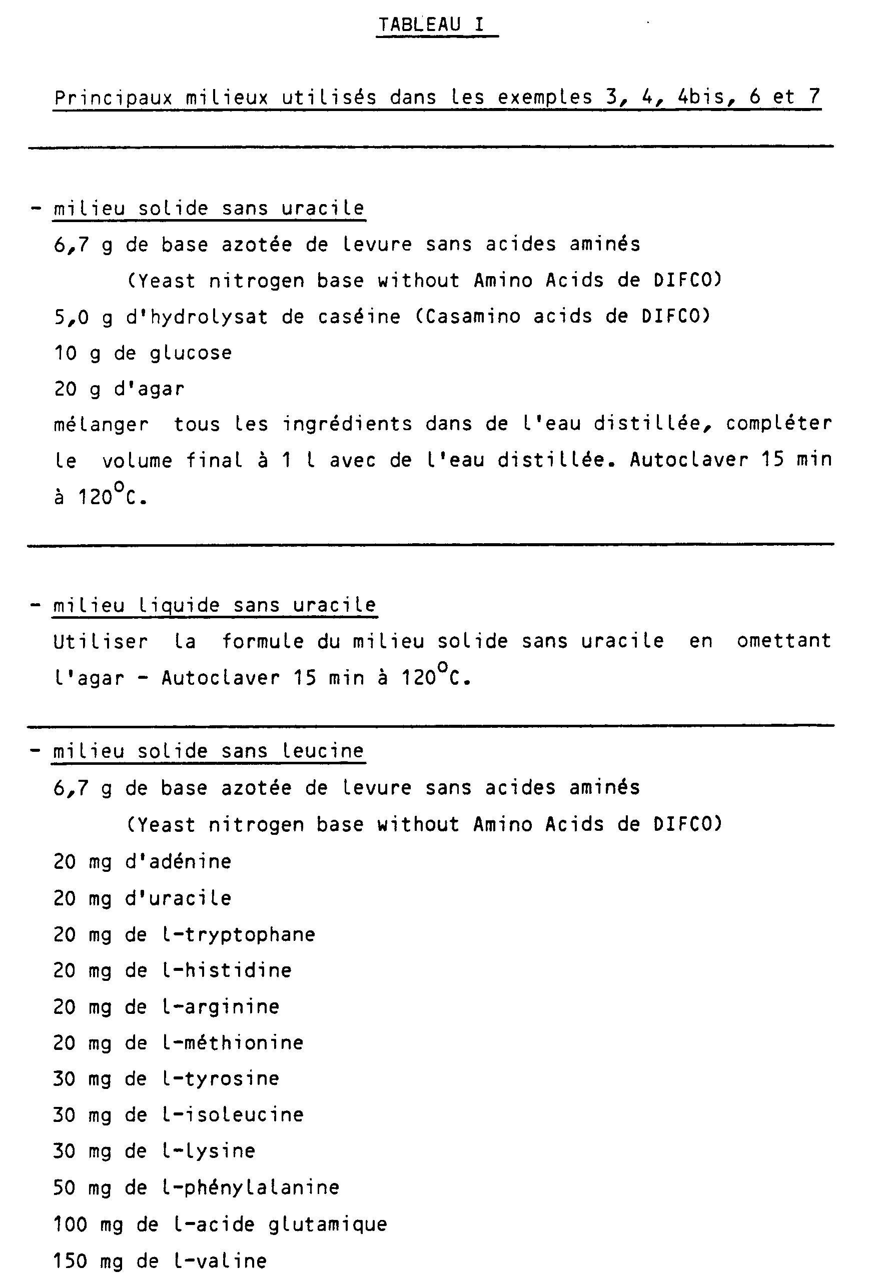

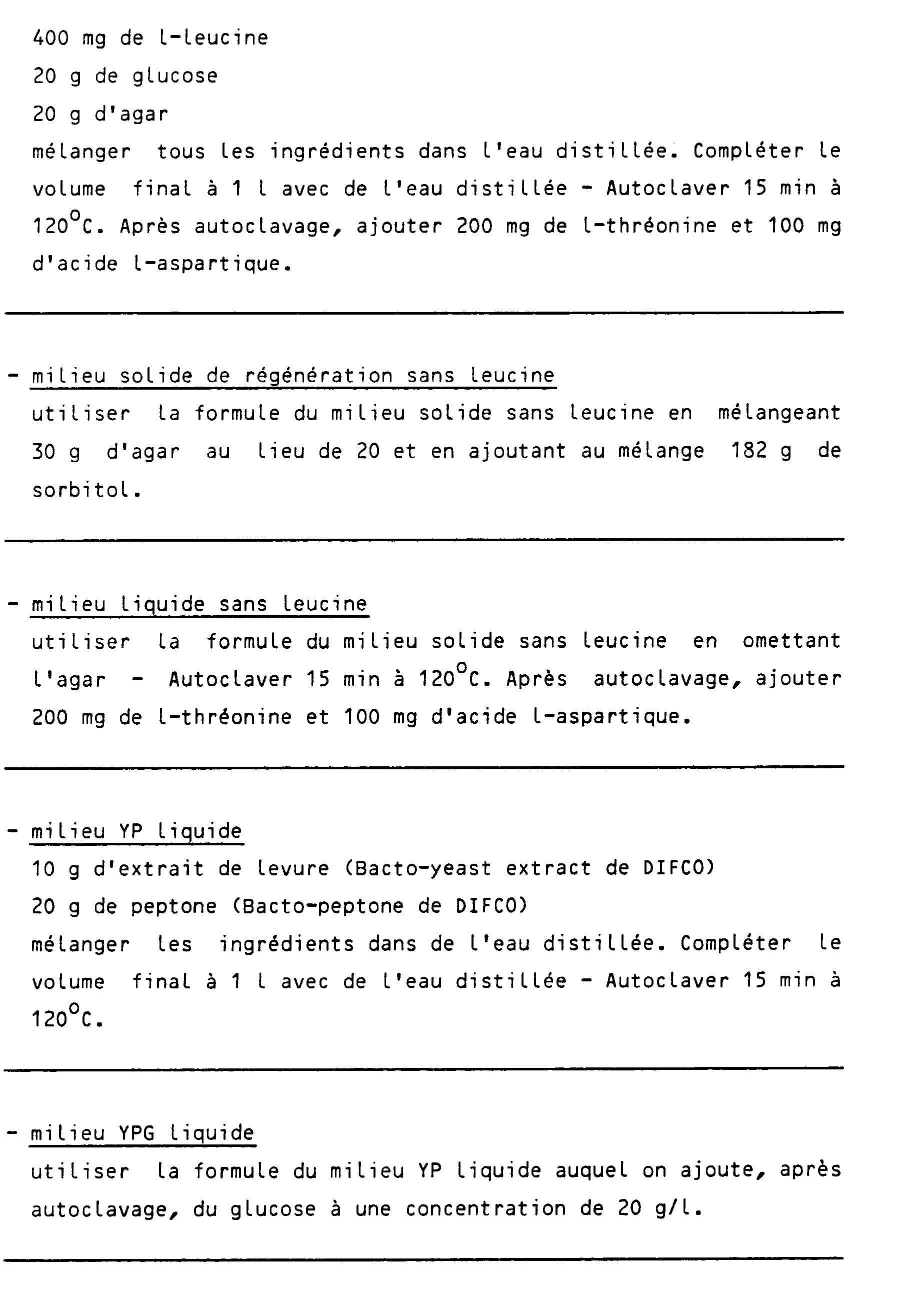

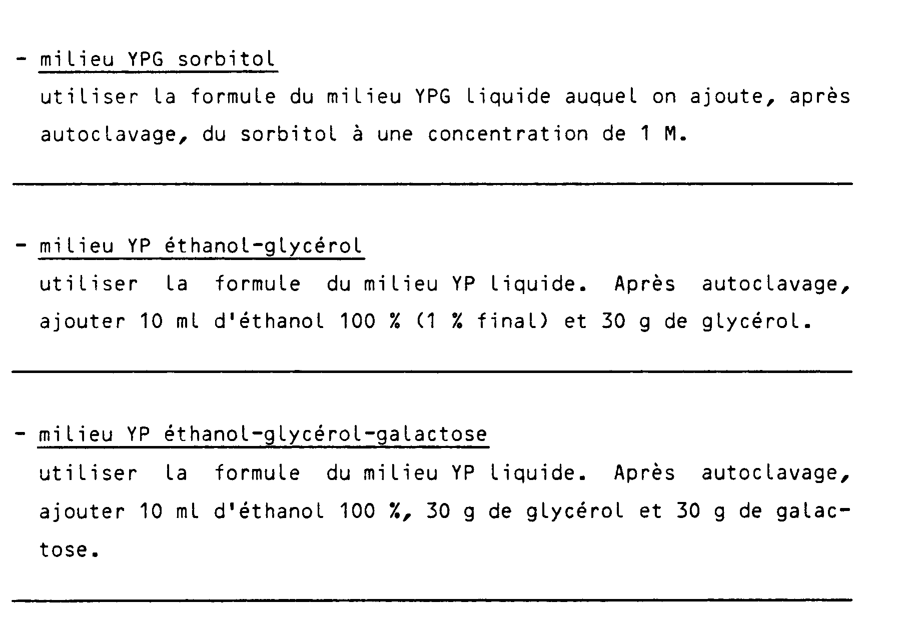

### EXEMPLE 4 : Expression de l'urate oxydase par les souches EMY761 pEMR469 (leu+) et EMY761 pEMR473 (leu+) (clones 1, 2 et 3) - Immunodétection par Western Blot, dosage de l'activité urate oxydase et des protéines solubles

### 1) Expression de l'urate oxydase :

### a) Souches transformées

Dans un premier temps, une colonie de chacune des souches EMY761 pEMR469 (leu+) et EMY761 pEMR473 (leu+) (clones 1, 2 et 3) a été mise en culture dans 25 ml de milieu liquide sans leucine (Cf. tableau I, exemple 3). Ceci a permis d'obtenir et de maintenir un nombre de copies de plasmides élevé en pratiquant la sélection pour la complémentation de la mutation leu2 par le gène LEU2 porté par les plasmides pEMR469 et pEMR473.

Après 22 h à 30 ° C sous agitation, les deux cultures ont été centrifugées pendant 10 min à 7 000 tr/min. Les culots ont été repris dans 10 ml d'eau distillée stérile et de nouveau centrifugés pendant 10 min à 7 000 tr/min. L'expression de l'urate oxydase a été induite en reprenant les cellules dans 20 ml de milieu YP éthanol-glycérol pour la souche EMY761 pEMR469 (leu⁺) et 20 ml de milieu YP éthanol-glycérol- galactose (Cf. tableau I, exemple 3) pour la souche EMY761 pEMR473 (leu⁺). Les cultures ont été replacées à 30 ° C sous agitation pendant 27 h.

### c) Souche témoin

La souche EMY761 non transformée, c'est-à-dire sans plasmide, a été cultivée comme ci-dessus avec comme différence que la première culture a eu lieu dans le milieur YPG liquide. Elle a subi d'une part l'induction dans 10 ml de milieu liquide YP éthanol-glycérol et, d'autre part, l'induction dans 10 ml de milieu YP éthanol-glycérolgalactose.

### 2) Préparation des échantillons :

a) Les cellules cultivées en 1a), 1 b) et 1c) ont été centrifugées et le surnageant a été éliminé. Les culots ont été repris dans 10 ml d'eau distillée et centrifugés pendant 10 min à 7 000 tr/min. Les culots ainsi lavés ont été repris dans environ 1 ml de tampon triéthylèneamine TEA de pH 8,9. Environ 300 µl de cellules ainsi reprises ont été lysées en présence de billes de verre (de 400 à 500 µm de diamètre) représentant environ la moitié du volume final. Ce mélange a été vortexé vigoureusement pendant 1 min, 4 fois, les échantillons étant placés pendant 30 s dans la glace entre chaque broyage. Le liquide a été retiré des tubes avec une pipette pasteur et transféré dans un microtube. Les billes de verre ont été lavées 1 fois avec environ 200 ul de tampon TEA de pH 8,9. Les billes ont été vortexées pendant 1 min, 1 fois, et le liquide a été retiré à la pipette pasteur pour être ajouté au lysat précédent. Le lysat a été ensuite centrifugé dans un microtube pendant 5 min à 7 000 tr/min. Le surnageant a été précautionneusement retiré et conservé à -20 ° C pour le Western Blot, le dosage de l'activité urate oxydase et le dosage des protéines totales solubles. Le culot des cellules lysées a été conservé séparément à -20 _{°} C pour le Western Blot (Cf. 3) ci-dessous).

D'autre part, des prélèvements des cultures réalisées en 1a) et 1 b) ont été réalisés comme suit avant l'induction: 2 ml de culture ont été centrifugés pendant 10 min à 7 000 tr/min. Les culots ont été repris dans 500 ul d'eau distillée et de nouveau centrifugés pendant 5 min à 7 000 tr/min. Les culots ont été repris dans environ 200 ul de tampon TEA de pH 8,9 et lysés comme ci-dessus en présence de billes de verre. Les surnageants et les culots des cellules lysées ont été conservés à -20 ° C séparément. Le dosage de l'activité oxydase et le dosage des protéines totales solubles ont été effectués sur les surnageants.

### 3) Immunodétection de l'urate oxydase par Western Blot :

### a) Mode opératoire

Les culots et les surnageants des différents échantillons ont été soumis à un Western Blot, technique bien connue de l'homme de l'art, qui comprend les étapes suivantes :
- solubilisation du culot par ébullition pendant 10 min dans un tampon dénommé tampon de charge constitué de tris-HCI 0,125 M pH 6,8 SDS 4 %, bleu de bromophénol 0,002 %, glycérol 20 %, β-mercaptoéthanol 10 % (selon le protocole décrit par LAEMMLI (U.K. LAEMMLI, Nature, 227 (1970), 680-685)), (étape mise en oeuvre uniquement pour les culots),
- séparation électrophorétique des différentes protéines contenues dans le solubilisat selon le protocole décrit par LAEMMLI (U.K. LAEMMLI, Nature, 227 (1970), 680-685),
- transfert desdites protéines contenues dans le gel sur un filtre de nitrocellulose (selon la technique de H. TOWBIN et al. Proc. Natl. Acad. Sci. USA 76 (1979) 4350-4354),

L'immunodétection, réalisée selon la technique de BURNETTE (W.W. BURNETTE Ana. Biochem. 112 - (1981) 195-203), implique successivement :
. Le rinçage du filtre de nitrocellulose pendant 10 min avec un tampon A (tris-HCI 10 mM, NaCI 170 mM, KCI 1 mM).
. La mise en contact du filtre de nitrocellulose pendant 30 min à 37 ° C avec un tampon B (tampon A additionné de sérum-albumine bovine à raison de 3 g pour 100 ml).
. La mise en contact du filtre de nitrocellulose pendant 1 h à 37_{°}C avec un immunsérum (des anticorps polyclonaux reconnaissant l'urate oxydase d'A. flavus).
. Le rinçage du filtre de nitrocellulose avec le tampon B.
. La mise en contact du filtre de nitrocellulose pendant 1 h à 37 ° C avec une solution de protéine G marquée à l'iode 125 à 0,1 microcurie/ml.
. Le rinçage du filtre avec le tampon A.
. Le séchage du filtre entre deux feuilles absorbantes.
. La mise en contact d'un film radiographique.
. La révélation du film.

### b) Résultats

On constate que les souches EMY761 pEMR469 (leu+) et EMY761 pEMR473 (leu+) (clone 1) produisent une protéine de poids moléculaire apparent d'environ 33 KDa qui est reconnue par les anticorps dirigés contre l'urate oxydase d'A. flavus (préparés chez le lapin selon des techniques bien connues de l'homme de l'art : Cf. VAITUKAITIS et al. (1981) "Methods in enzymology" Academic Press, New York, vol. 73, p. 46) et qui est absente de la souche témoin.

La comparaison entre les quantités de cette protéine pour les culots et les surnageants permet de déduire qu'environ 80 % de celle-ci est sous forme soluble dans le lysat.

### 4) Dosage de l'activité urate oxydase :

L'activité urate oxydase a été mesurée sur les surnageants des cellules lysées.

### a) Principe

On suit la transformation de l'acide urique en allantoïne par la diminution de l'absorbance à 292 nm. La réaction est la suivante :

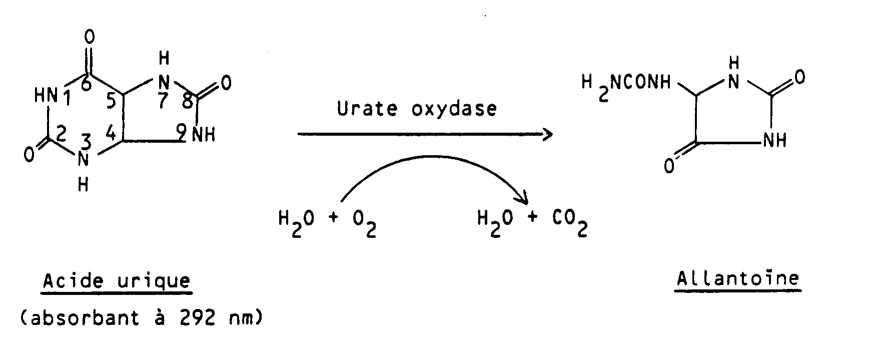

### b) Réactifs

a) Tampon TEA 0,05 M pH 8,9/EDTA
   - dissoudre 7,5 g de TEA (réactif pour analyse-Prolabo réf. 287.46.266) dans 400 ml d'eau distillée,
   - dissoudre 0,372 g de Complexon III (Merck-réf. 8418) dans 50 ml d'eau distillée,
   - réunir les deux solutions et compléter à 500 ml (solution 1),
   - ajuster le pH de cette solution à 8,9 par HCI 0,2N,
   - compléter à 1 000 ml avec de l'eau distillée (solution 2).
b) Acide urique Solution stock
   - dissoudre 100 mg d'acide urique (Carbiochem réf. 6671) dans 50 ml de la solution 1,
   - ajuster par HCI 0,2N à pH 8,9,
   - compléter à 100 ml par de l'eau distillée.

   La solution obtenue peut se conserver une semaine à 4 ° C.
c) Acide urique Solution Substrat
   - prélever 1,5 ml de solution stock d'acide urique (carbiochem réf. 6671) et diluer à 100 ml avec du tampon TEA(réactif pour analyse-Prolabo réf. 287.46.266).

   Cette solution doit être utilisée dans la journée.

### c) Mode opératoire

On introduit dans la cuve de quartz d'un spectrophotomètre réglé à 292 nm et thermostaté à 30 ° C les volumes suivants :
- 600 µl d'acide urique solution substrat (préchauffés à 30 ° C),
- 100 µl des surnageants ci-dessus auxquels ont été ajoutés 200 µl de TEA pH 8,9 (préchauffés à 30 ° C).

On mélange et on lit le changement de densité optique, ci-après parfois abrégé DO, toutes les 30 s pendant 5 min. On en déduit AE, variation de la densité optique par minute.

### d) Expression des résultats

L'activité A enzymatique urate oxydase exprimée en U/ml est calculée à partir de la mesure de AE à l'aide de la formule : 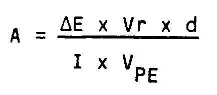 dans laquelle les symboles Vr, d, 1 et V_{PE} représentent respectivement le volume réactionnel (0,9 ml), le taux de dilution (2), le coefficient d'extinction de l'acide urique à 292 nm (12,5) et le volume de la prise d'essai (0,1 ml).

### 5) Dosage des protéines totales solubles dans les lysats :

Le kit protein assay de BIORAD a été utilisé pour doser les protéines totales présentes dans le surnageant des cellules lysées. Il est basé sur l'observation que l'absorbance maximum pour une solution acide de bleu brillant de Coomassie g-250 passe de 465 nm à 595 nm lorsque des protéines viennent s'y fixer (Cf. Reisner et al., Anal Biochem, 64, 509-(1975)).

### Mode opératoire

On introduit dans la cuve d'un spectrophotomètre réglé à 595 nm les volumes suivants :
- 10 µl d'échantillon auxquels ont été rajoutés 790 µl d'eau distillée
- 200 µl de "Dye reagent" concentré (Biorad).

On mélange et on lit la densité optique à 595 nm. Une gamme étalon avec des concentrations croissantes de BSA (Bovine Serum Albumine) a été réalisée ainsi. On lit la concentration inconnue des protéines totales des lysats sur la courbe étalon obtenue.

### 6) Résultats :

Les résultats obtenus sont rassemblés dans le tableau (II) ci-aprés. Celui-ci précise pour chaque souche le milieu de culture, la source de carbone et d'énergie de la culture, l'activité urate oxydase en U/ml, la quantité de protéines totales solubles en mg/ml et le pourcentage d'urate oxydase dans les protéines totales solubles. Ce dernier paramètre est calculé en supposant que l'activité spécifique de la protéine recombinante est identique à celle de l'urate oxydase obtenue à partir d'A. flavus : 30 U/mg.

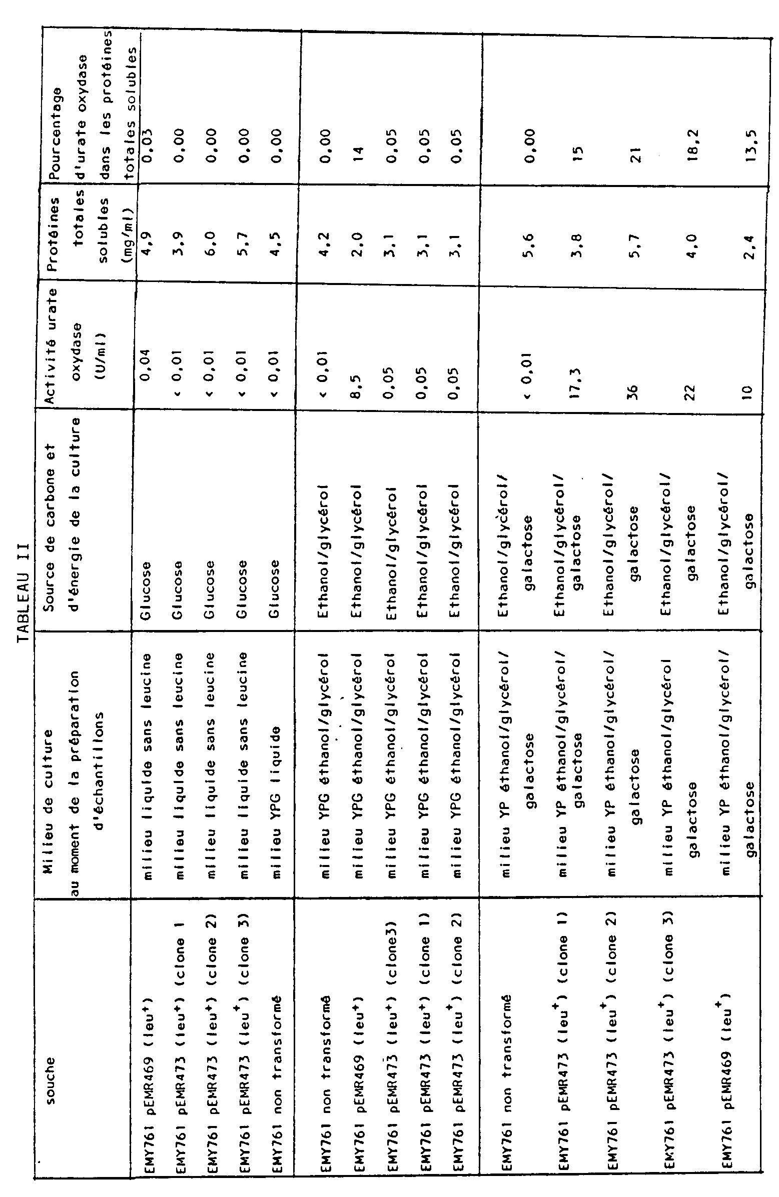

Ce tableau montre :
a) en présence de glucose, le niveau ("réprimé") d'urate oxydase est non détectable dans le cas du promoteur artificiel de l'invention (souche EMY761 pEMR473 (leu+) (clone 1, 2 ou 3)) alors qu'il l'est dans le cas du promoteur ADH₂ (souche EMY761 pEMR469 (leu⁺)). Le promoteur artificiel permet donc, en présence de glucose, une meilleure répression que le promoteur ADH₂.
b) en absence de glucose, mais en présence d'éthanol/glycérol, le niveau d'urate oxydase est important pour le promoteur ADH₂ (environ 14 % des protéines totales solubles) et faible mais détectable pour le promoteur artificiel.
c) en absence de glucose mais en présence d'éthanol/glycérol/galactose, le niveau d'urate oxydase garde une valeur peu différente de celle du cas précédent pour le promoteur ADH₂ (environ 13,5 % des protéines totales solubles), mais atteint une valeur élevée (environ 18 % des protéines totales solubles) pour le promoteur artificiel.

Le promoteur artificiel de l'invention permet donc une production de protéine recombinante à un niveau élevé et présente trois niveaux d'expression :
- niveau nul a)
- niveau de fond b)
- niveau maximal c)

### EXEMPLE 4bis : Expression en erlenmeyer de l'ADNc de l'urate oxydase par les souches EMY761 pEMR515 (leu⁺),EMY500 pEMR515 (leu+) et GRF18 pEMR515 (leu⁺)

Une colonie de chacune des trois souches ci-dessus a été mise en culture dans 20 ml de milieu liquide sans leucine.

Après une nuit à 30 ° C sous agitation, les trois cultures ont été centrifugées pendant 10 min à 7 000 tr/min. Les culots cellulaires ont été repris dans 10 ml d'eau distillée stérile et de nouveau centrifugés pendant 10 min. L'expression de l'urate oxydase a été induite en reprenant les cellules dans 20 ml de milieu YP éthanol-glycérol-galactose (Cf. tableau I, exemple 3). Les cultures ont été replacées à 30 _{°} C sous agitation pendant environ 20 h. En témoin, une culture de chaque souche hôte, non transformée, a été effectuée.

Les cellules de chacune des six cultures sont reculotées par centrifugation et le surnageant est éliminé. Les culots ont été repris dans 10 ml d'eau distillée et centrifugés pendant 10 min à 7 000 tr/min. Les culots ainsi lavés ont été repris dans environ 1 ml de tampon TEA de pH 8,9 et le broyage ainsi que l'élimination des particules par centrifugation ont été réalisés comme décrit dans l'exemple 4, 2). Le surnageant de chaque culture sert comme précédemment à un dosage de l'urate oxydase et des protéines totales. Les principaux résultats obtenus sont rassemblés dans le tableau III ci-après :

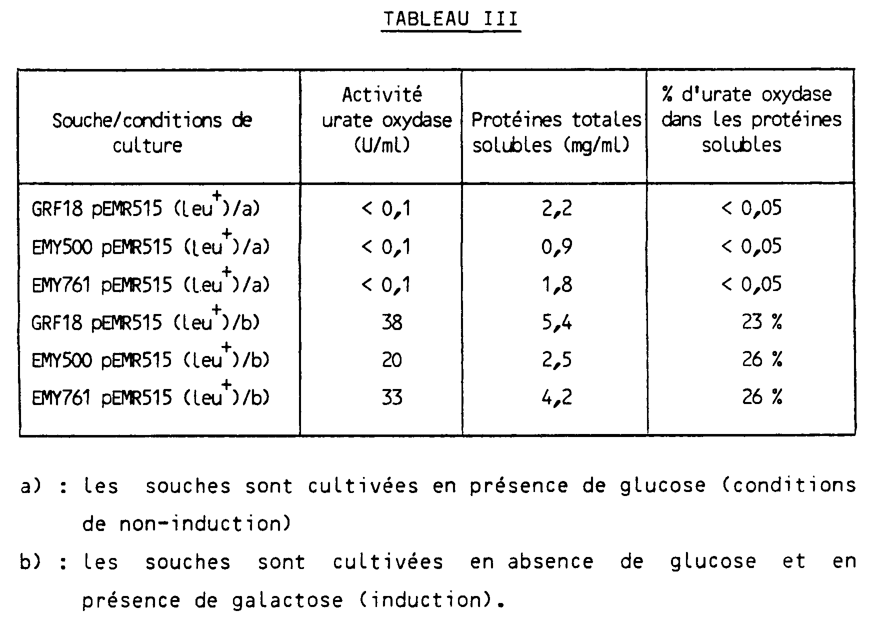

Le promoteur selon l'invention permet donc un niveau d'expression élevée de l'urate oxydase dans trois souches non isogéniques.

### EXEMPLE 5 : Construction de deux vecteurs d'expression de la β-galactosidase dans la levure : le plasmide pEMR429 porteur d'un promoteur ADH₂ et le plasmide pEMR437 porteur du promoteur artificiel de l'invention

La stratégie mise en oeuvre fait appel à des fragments obtenus à partir de plasmides préexistants accessibles au public et à des fragments préparés par voie de synthèse selon les techniques maintenant couramment utilisées. Les techniques de clonage employées sont celles décrites par T. MANIATIS EF, FRITSCH et J. SAMBROOK dans "Molecular Cloning, a laboratory manual" (Cold Spring Harbor Laboratory, 1984). La synthèse des oligonucléotides est réalisée à l'aide d'un synthétiseur d'ADN Biosearch 4 600.

### 1) Construction du plasmide pEMR429 :

Le plasmide pEMR414 (Cf. figure 5) a été soumis à une digestion complète par l'enzyme de restriction BamHl. Le site BamHl, localisé entre les séquences promotrices (ADH₂) et terminatrices(PGK) est unique. L'ADN linéaire du plasmide est purifié par élution d'un gel d'agarose après électrophorèse. Le plasmide pMC1403 (réf. Casadaban et al. (1980), J. Bacteriol., 143, 971-980) a été soumis à une digestion complète par les enzymes BamHl et EcoRI, laquelle a permis de libérer un fragment d'ADN BamHl-EcoRl d'environ 3 Kb qui contient l'essentiel (partie amont) de la séquence codant pour la β-galactosidase d'E. coli. La séquence complète a été reconstituée à l'aide du fragment synthétique de séquence suivante :

5' EcoRI AATTTCAGCTGAGCGCCGGTCG
AGTCGACTCGCCGCCACC

CTACCATTACCAGTTGGTCTGGT
GATGGTAATGGTCAACCAGACCA

GTCAAAAATAATAATAAG
CAGTTTTTATTATTATTCCTAG
BamHI

Le fragment BamHl-EcoRl provenant de la double digestion de pMC1403 est ligué en EcoRI avec le fragment synthétique ci-dessus.

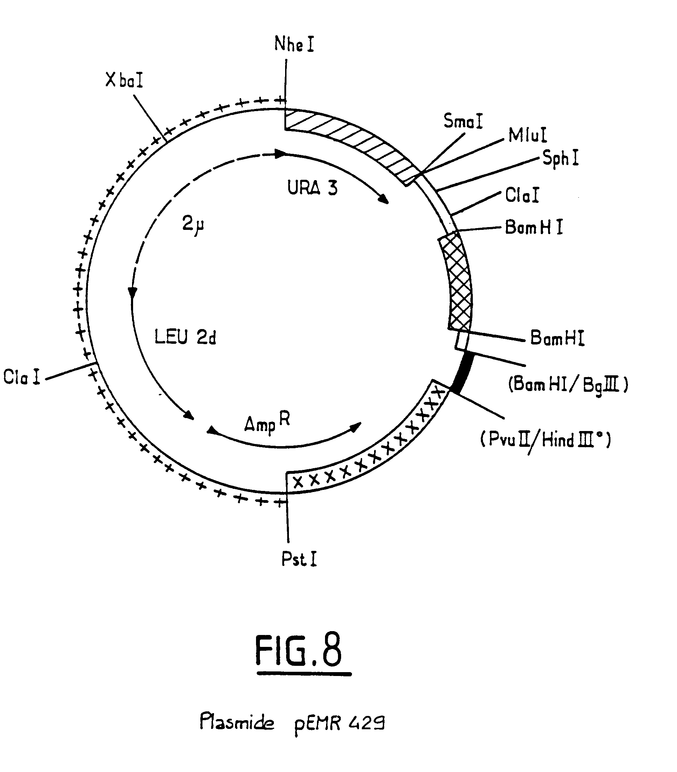

Le fragment BamHl-BamHl obtenu est ensuite ligué à l'ADN linéaire du plasmide pEMR414 digéré par BamHl de façon à obtenir le plasmide pEMR429, représenté sur la figure 8, dans laquelle les symboles ont la même signification que dans la figure 5, les fragments BamHl-EcoRl et IcoRI-BamHI introduits étant représentés par

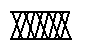 Dans ce plasmide, la séquence codant pour la β-galactosidase est sous le contrôle du promoteur ADH₂ décrit dans l'exemple 2.

### 2) Construction du plasmide pEMR461 :

Le plasmide pEMR429 a été soumis à une digestion complète par les enzymes Mlul et Sphl. Le grand fragment Mlul-Sphl contenant le gène de la β-galactosidase a ensuite été ligué au fragment synthétique, dont la séquence donnée ci-après comporte les séquences d'activation amont (Upstream Activation Sequence : UAS) du promoteur du gène GAL7 de S. cerevisiae et des extrémités cohésives Mlul et Sphl.

M
l
u
I

CGCGTCTATACTTCGGAGCACTGTTGAGCGAAGGCTCATTAGATATATTTTCTGTCAT
--+---------+---------+---------+---------+---------+-----
AGATATGAAGCCTCGTGACAACTCGCTTCCGAGTAATCTATATAAAAGACAGTA

TTTCCTTAACCCAAAAATAAGGGAGAGGGTCCAAAAAGCGCTCGGACAACTGTTGACCGT
----+---------+---------+---------+---------+---------+-----
AAAGGAATTGGGTTTTTATTCCCTCTCCCAGGTTTTTCGCGAGCCTGTTGACAACTGGCA

GATCCGAAGGACTGGCTATACAGTGTTCACAAAATAGCCAAGCTGAAAATAATGTGTAGC
----+---------+---------+---------+---------+---------+-----
CTAGGCTTCCTGACCGATATGTCACAAGTGTTTTATCGGTTCGACTTTTATTACACATCG

S
p
h
I
CTTTAGCTATGTTCAGTTAGTTTGGCATG
----+---------+---------+-----
GAAATCGATACAAGTCAATCAAACC

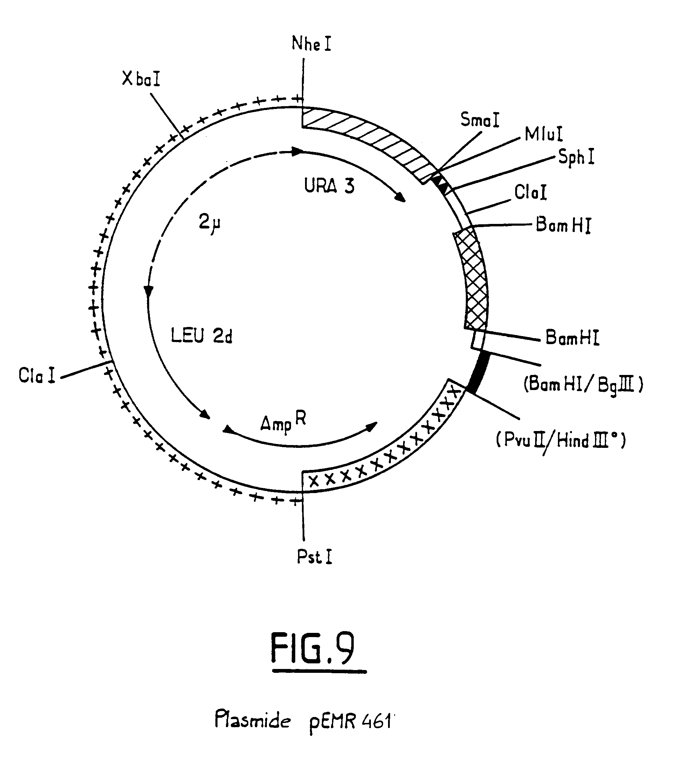

Le plasmide pEMR461 ainsi obtenu est représenté sur la figure 9, dans laquelle les symboles ont la même signification que dans la figure 8, le nouveau fragment Mlul-Sphl introduit étant symbolisé par 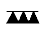

Dans ce plasmide, la séquence codant pour la β-galactosidase est sous le contrôle du promoteur artificiel de l'invention décrit dans l'exemple 2.

### EXEMPLE 6 : Transformation de la souche de S. cerevisiae DBY746 par les plasmides pEMR429 et pEMR461

Transformation avec sélection pour prototrophie de l'uracile :
Une colonie de la souche DBY746 qui est (Mata, his3, leu2, ura3, trp1, cyh^{R}) (ROSE et al. (1981), PNAS USA, 78, 2460-2464) a servi à ensemencer 100 ml d'un milieu appelé milieu YPG liquide (CF; tableau 1 de l'exemple 3). Lors de l'obtention d'une densité cellulaire de 10⁷ cellules par ml, les cellules ont été traitées à l'acétate de lithium 0,2 M pour la transformation selon une technique bien connue de l'homme de l'art, décrite par ITO et al. (ITO et al., 1983, J. Bacteriology 153, 163-168).

Les cellules DBY746 ont été transformées en parallèle avec environ 1 µg de chacun des plasmides pEMR429 et pEMR461. Les cellules transformées sont sélectionnées pour le caractère d'auxotrophie d'uracile (ura⁺) sur un milieu appelé milieu solide sans uracile, (Cf. tableau 1 de l'exemple 3). On a ainsi retenu un transformé DBY746 pEMR429 (ura⁺) et un transformé DBY746 pEMR461 (ura⁺).

### EXEMPLE 7 : Production de β-galactosidase à l'aide des souches DBY746 pEMR429 (ura⁺) et DBY746

### pEMR461 (ura⁺)

### 1) Expression de la β-galactosidase :

Une colonie transformée DBY746 pEMR429 (ura⁺) et une colonie transformée DBY746 pEMR461 (ura⁺) ont servi à ensemencer chacune 20 ml de milieu liquide sans uracile auquel on a préalablement ajouté du tryptophane (10 mg/I). Après une nuit à 30 ° C sous agitation, on ajoute 1 % de glucose et on laisse la culture se poursuivre pendant 4 h. On vérifie alors qu'il y a encore du glucose dans les cultures. On prélève une fraction aliquote pour doser la β-galactosidase.

Après une nuit à 30 ° C sous agitation, les deux cultures ont été centrifugées pendant 10 min à 7 000 tr/min. Les culots ont été repris dans 10 ml d'eau distillée stérile et de nouveau centrifugés pendant 10 min à 7 000 tr/min. L'expression de β-galactosidase a été induite en reprenant les cellules dans 20 ml de milieu YP éthanol-glycérol (Cf. tableau I, exemple 3) pour la souche DBY746 pEMR429 (ura⁺) et 20 ml de milieu YP éthanol-glycérol-galactose (Cf. tableau I, exemple 3) pour la souche DBY746 pEMR461 (ura⁺). Les cultures ont été replacées à 30 ° C sous agitation pendant une nuit.

### 2) Préparation des échantillons et dosage :

Les cellules cultivées ci-dessus ont été centrifugées et le surnageant a été éliminé. Les culots ont été repris dans 10 ml d'eau distillée et centrifugés pendant 10 min à 7 000 tr/min. Les culots ainsi lavés ont été repris dans environ 1 ml de tampon de dosage de la β-galactosidase (EDTA : 2 x 10-³ M ; Na₂HP0₄ 7 x 10-² M ; NaH2PO4 3 x 10-² M ; MgS0₄ 10-³ M ; MnS0₄ 2 x 10-³ M). Environ 300 µl de cellules ainsi reprises ont été lysées en présence de billes de verre (de 400 à 500 µm de diamètre) représentant environ la moitié du volume final. Ce mélange a été vortexé vigoureusement pendant 1 min, 4 fois, les échantillons étant placés pendant 30 s dans la glace entre chaque broyage. Le liquide a été retiré des tubes avec une pipette pasteur et transféré dans un microtube. Les billes de verre ont été lavées 1 fois avec environ 200 µl de tampon TEA de pH 8,9. Les billes ont été vortexées pendant 1 min, 1 fois, et le liquide a été retiré à la pipette pasteur pour être ajouté au lysat précédent. Le lysat a été ensuite centrifugé dans un microtube pendant 5 min à 7 000 tr/min. Le surnageant a été précautionneusement retiré et conservé à -20 ° C pour le Western Blot, le dosage de l'activité urate oxydase et le dosage des protéines totales solubles. Le culot des cellules lysées a été conservé séparément à -20 ° C.

L'activité β-galactosidase a été dosée selon la technique de PARDEE (PARDEE et al., J. Mol. B. (1959), 1, 1656-178).

Les protéines totales solubles ont été d'autre part dosées à l'aide du kit protein essay de BIORAD, comme décrit dans l'exemple 4.

Les résultats obtenus sont rassemblés dans le tableau IV ci-après :

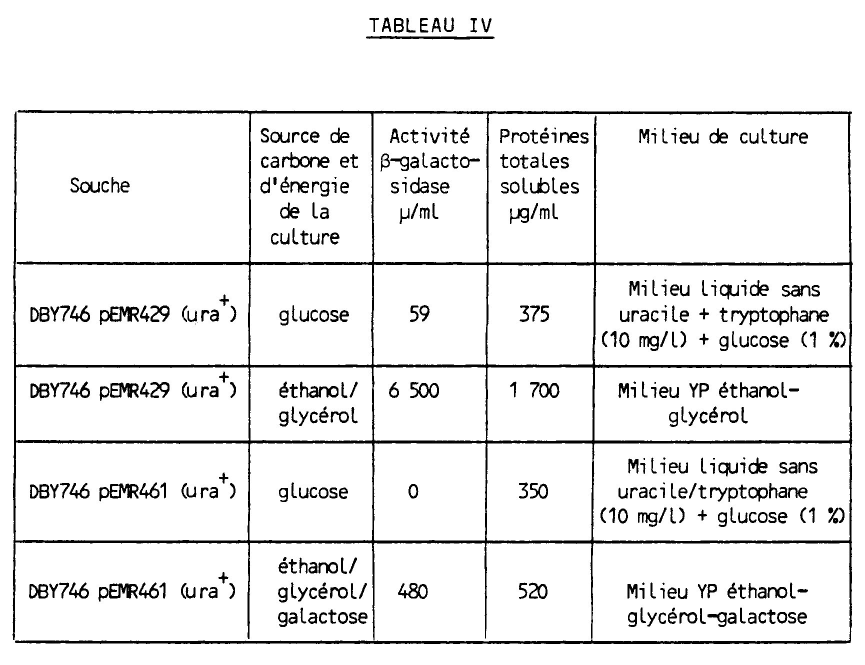

Ce tableau montre :
- en présence de glucose, la souche EMY746 pEMR429 (ura⁺) produit un peu de β-galactosidase alors que l'on ne détecte pas cette protéine pour la souche DBY746 pEMR461 (ura⁺). Le promoteur artificiel de l'invention permet donc une meilleure répression que le promoteur ADH₂.
- en conditions d'induction, le promoteur artificiel conduit à un niveau d'expression élévé de β-galactosidase quoique, dans ces conditions, plus faible que le niveau obtenu avec le promoteur ADH₂.

M
l
u
I
CGCGTCTATACTTCGGAGCACTGTTGAGCGAAGGCTCATTAGATATATTTTCTGTCAT
---+---------+---------+---------+---------+---------+-----
    AGATATGAAGCCTCGTGACAACTCGCTTCCGAGTAATCTATATAAAAGACAGTA

TTTCCTTAACCCAAAAATAAGGGAGAGGGTCCAAAAAGCGCTCGGACAACTGTTGACCGT
---+---------+---------+---------+---------+---------+------
AAAGGAATTGGGTTTTTATTCCCTCTCCCAGGTTTTTCGCGAGCCTGTTGACAACTGGCA

GATCCGAAGGACTGGCTATACAGTGTTCACAAAATAGCCAAGCTGAAAATAATGTGTAGC
---+---------+---------+---------+---------+---------+------
CTAGGCTTCCTGACCGATATGTCACAAGTGTTTTATCGGTTCGACTTTTATTACACATCG

S
p
h
I
CTTTAGCTATGTTCAGTTAGTTTGGCATGCCTATCACATATAAATAGA
---+---------+---------+---------+---------+----
GAAATCGATACAAGTCAATCAAACCGTACGGATAGTGTATATTTATCT

GTGCCAGTAGCGACTTTTTTCACACTCGAGATACTCTTACTACTGCTCTCTTGTTGTTTT
---+---------+---------+---------+---------+---------+------
CACGGTCATCGCTGAAAAAAGTGTGAGCTCTATGAGAATGATGACGAGAGAACAACAAAA

TATCACTTCTTGTTTCTTCTTGGTAAATAGAATATCAAGCTACAAAAAGCATACAATCAA
---+---------+---------+---------+---------+---------+------
ATAGTGAAGAACAAAGAAGAACCATTTATCTTATAGTTCGATGTTTTTCGTATGTTAGTT

C
l
a
I
CTATCAACTATTAACTATAT
---+---------+------
GATAGTTGATAATTGATATAGC

### EXEMPLE 8 : Construction de deux vecteurs d'expression et de sécrétion de la cytokine humaine gro-,8 dans la levure : les plasmides pEMR 575 et pEMR 583, porteurs du promoteur artificiel de l'invention :

Les exemples décrits précédemment concernent des protéines dont la localisation est intracellulaire. Or, il est connu que la levure peut sécréter des protéines recombinantes dans le milieu de culture. L'utilisation du chemin métabolique conduisant à la sécrétion de la protéine a plusieurs avantages importants.
1 - il permet de récupérer dans le surnageant de culture un produit raisonnablement pur et correctement maturé.
2 - il permet de faire bénéficier à la protéine des modifications associées au chemin de sécrétion comme la formation de ponts disulfures, la glycosylation, etc.

Il existe plusieurs protéines ou polypeptides naturellement sécrétés par la levure. Dans la plupart des cas connus, ces protéines sont synthétisées sous forme d'un précurseur plus long dont la séquence NH₂- terminale est décisive pour l'entrée dans le chemin métabolique conduisant à la sécrétion. Ces séquences NH₂-terminales peuvent, dans certains cas, être utilisées pour la sécrétion de protéines hétérologues. Parmi ces séquences, il est connu que l'on peut utiliser le système pré-pro de la phéromone alpha. La phéromone sexuelle alpha de levure est un peptide de 13 aminoacides qui est sécrété dans le milieu de culture par les levures S. cerevisiae de type sexuel Mata.

Le facteur alpha arrête les cellules de type sexuel opposé (Mata) en phase G 1 et induit les changements biochimiques et morphologiques nécessaires à la conjugaison des 2 types de cellules - Kurjan, J. et Herskowitz, I. (1982) Cell, 30, 933-943 ont cloné le gène structural du facteur alpha et ont déduit de la séquence de ce gène que ce facteur de 13 aminoacides est synthétisé sous forme d'une pré-pro protéine précurseur de 165 aminoacides. Le précurseur contient une séquence hydrophobe aminoterminale de 22 aminoacides suivie d'une séquence de 61 aminoacides contenant 3 sites de glycosylation suivis enfin de 4 copies du facteur a. Ces 4 copies sont séparées par des séquences "spacer" et la protéine mature est libérée à partir du précurseur grâce aux activités enzymatiques suivantes :
1 - une endopeptidase du type Cathepsine B qui coupe au niveau de l'extrémité carboxy terminale des dipeptides Lys-Arg (produit du gène KEX2 appelé yscF).
2 - une exopeptidase de type carboxypeptidase (produit du gène KEX1) qui coupe les résidus basiques au niveau de l'extrémité carboxy terminale des peptides excisés.
3 - une dipeptidylaminopeptidase (dite A) qui enlève les doublets Glu-Ala et Asp-Ala (produit du gène STE 13).

Un premier exemple de protéine sécrétée par ce système et qui utilise le promoteur de l'invention est la cytokine humaine gro-β . L'ADNc de cette protéine, appelée soit gro-,8 (S. Haskill, et al, 1990 Proc. Natl. Acad. Sci. USA, 87, 7732-7736), soit MIP-2a (P. Tekamp-Olson et al, 1990 J. Exp., 172, 911-919) a été cloné et séquencé récemment. Gro-,8 appartient à une famille de cytokines dont les membres apparaissent être impliqués dans la modulation de la réponse inflammatoire et dans des activités du type facteur de croissance.

La demanderesse a testé l'utilisation du promoteur pour la sécrétion de gro-,8 par S. cerevisiae. Pour cela, elle a substitué à la séquence signal naturelle du gro-,8 la séquence pré-pro de la phéromone alpha et placé le précurseur du gro-β derrière le promoteur de l'invention.

1 - Construction du plasmide pEMR 530 (vecteur de clonage) :
Le plasmide pEMR 473 décrit plus haut (exemple 2.2)-fig. 7 a été digéré par les enzymes Xhol et Sall et le grand fragment, dénommé ci-après fragment E, isolé. Ce grand fragment comprend les séquences du gène URA 3, l'origine de réplication et le locus STB du 2 µ, le gène de résistance à l'ampicilline Amp^{R}, l'origine de réplication du plasmide pBR322 et les séquences UAS du promoteur du gène GAL7 de S. cerevisiae ainsi que le terminateur du gène PGK.

Une séquence oligonucléotidique double brin d'environ 400 paires de base, appelée fragment F, a été synthétisée sous forme d'un fragment Xhol-Sall. Cette séquence apporte la région TATA et la région d'initiation du promoteur ADH2 qui se prolonge par une séquence synthétique précédant le début de la région pré-pro de la phéromone alpha. La séquence de cette région pré-pro de la phéromone alpha diffère de celle décrite par Kurjan et Herskowitz 1982, Cell, 30, 933-943 par l'introduction d'un site Hind III par la mutation silencieuse du codon TCT - correspondant à la sérine 81 du précurseur de la phéromone - en AGC. L'ensemble de la séquence du fragment est donné ci-aprés :

X
h
o
I
TCGAGATACTCTTACTACTGCTCTCTTGTTGTTTTTATCACTTCTTGTTTC
-+---+------+---------+---------+---------+---------
TATGAGAATGATGACGAGAGAACAACAAAAATAGTGAAGAACAAAG

TTCTTGGTAAATAGAATATCAAGCTACAAAAAGCATACAATCAACTATCAATCAGATCTA
+---------+---------+---------+---------+---------+---------
AAGAACCATTTATCTTATAGTTCGATGTTTTTCGTATGTTAGTTGATAGTTAGTCTAGAT

ATATTAATAAAAAATGAGATTTCCTTCAATTTTTACTGCAGTTTTATTCGCAGCATCCTC
+---------+---------+---------+---------+---------+---------
TATAATTATTTTTTACTCTAAAGGAAGTTAAAAATGACGTCAAAATAAGCGTCGTAGGAG

CGCATTAGCTGCTCCAGTCAACACTACAACAGAAGATGAAACGGCACAAATTCCGGCTGA
+---------+---------+---------+---------+---------+---------
GCGTAATCGACGAGGTCAGTTGTGATGTTGTCTTCTACTTTGCCGTGTTTAAGGCCGACT

AGCTGTCATCGGTTACTTAGATTTAGAAGGGGATTTCGATGTTGCTGTTTTGCCATTTTC
+---------+---------+---------+---------+---------+---------
TCGACAGTAGCCAATGAATCTAAATCTTCCCCTAAAGCTACAACGACAAAACGGTAAAAG

CAACAGCACAAATAACGGGTTATTGTTTATAAATACTACTATTGCCAGCATTGCTGCTAA
+---------+---------+---------+---------+---------+---------
GTTGTCGTGTTTATTGCCCAATAACAAATATTTATGATGATAACGGTCGTAACGACGATT

H
i
n
d
I
I
I
AGAAGAAGGGGTAAGC TTGCATGCCTGCAGG
+---------+---------+---------+---------+
TCTTCTTCCCCATT CGAACGTACGGACGTCCAGCT

S
a
l
I

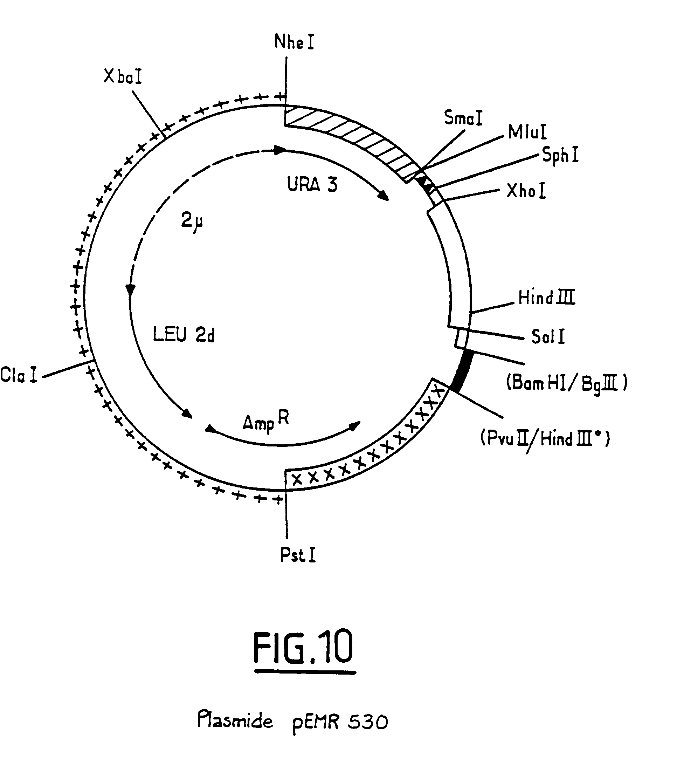

Les fragments E et F ont été ligués de manière à obtenir le plasmide pEMR 530, représenté sur la figure 10, dans laquelle les symboles ont la même signification que dans la figure 7, le nouveau fragment Xhol-Sall (fragment F) introduit étant représenté par : 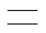

Ce plasmide comprend le promoteur artificiel de l'invention dont la séquence a été donnée à l'exemple 2. 2 - Construction du plasmide pEMR 583 (plasmide d'expression de la cytokine humaine gro-β).

Le plasmide pEMR 530 a été soumis à une digestion complète par les enzymes Nhel et Hind III. Le petit fragment Nhel-Hind III contenant le promoteur artificiel et la région pré-pro de la phéromone alpha ainsi que le gène URA3 a été purifié (fragment dénommé ci-après G).

Le plasmide pEMR 473 a été soumis à une digestion complète par les enzymes de restriction Nhel et BamHl. Le grand fragment, (appelé ci-après fragment H), comprenant l'origine de réplication et le locus STB du 2 µ, le gène LEU2d, le gène de résistance à l'ampicilline, l'origine de pBR322 et le terminateur du gène PGK a été purifié.

L'ADNc de gro-β a été cloné et séquencé selon la méthode décrite par Tekamp-Olson et al, référence ci-dessus. La séquence de l'ADNc de gro-β (décrite en détail dans cette référence - en particulier figure 2) présente un site EcoRI (séquence : 5'-GAATTC) qui couvre le codon ATT (Isoleucine à la position + 18 de la séquence mature du gro-β, Tekamp-Olson et al, référence ci-dessus) et déborde sur les deux codons flanquants GGA et CAC. L'ADNc cloné de gro-β se termine en 3' par une queue de polyA, flanquée d'un site de restriction BamHl.

Le fragment EcoRI-BamHl comprenant la majeure partie de la séquence codante du gro-β suivie de la séquence 3' correspondant à la fin non traduite du mRNA flanqué de la queue polyadénylée a été isolé selon les techniques usuelles et décrites dans Maniatis et al. (Réf. citée ci-dessus). Le fragment est ci-après appelé fragment I.

On a préparé d'autre part un fragment synthétique Hindlll-EcoRI contenant la fin de la région pré-pro de la phéromone alpha (correspondant aux résidus Ser Leu Asp Lys Arg) et le début de la séquence codant pour la protéine mature du gro-β. La séquence de ce fragment, appelé fragment J, est donnée ci-après. On remarquera que la séquence correspondant au début de l'ADNc du gro-β a été modifiée par rapport à la séquence de l'ADNc décrit par Tekamp-Olson et al, référence ci-dessus, de façon que les codons utilisés soient parmi les plus fréquemment utilisés par S. cerevisiae (Cf. Sharp et al. 1986 Nucleic Acids Research, vol.14, 13, 5125-5143). Hind III

AGCTTGGATAAAAGAGCGCCTTTGGCTACTGAATTGAGATGTCAATGTTTGCAAACCTTGCAAGG
ACCTATTTTCTCGCGGAAACCGATGACTTAACTCTACAGTTACAAACGTTTGGAACGTTCCTTAA
EcoRI

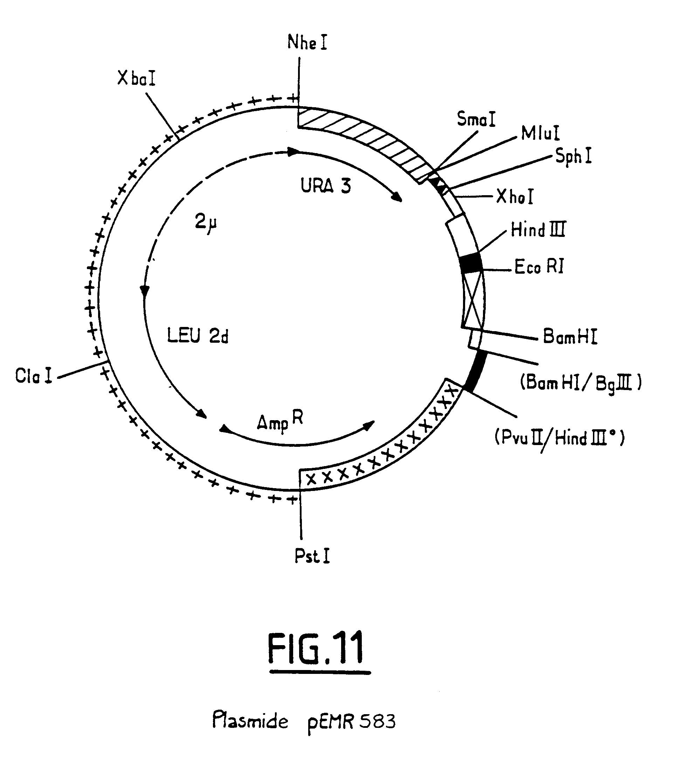

Les fragments G, H, 1 et J ont été ligués de manière à obtenir le plasmide pEMR 583, représenté sur la figure 11, dans laquelle les symboles ont la même signification que dans les figures 7 et 10, le fragment E étant représenté par 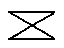 et le fragment F par 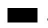 .

Les symboles nucléotidiques et peptidiques du début de la protéine mature de gro-β, et de la fin de la région pré-pro de la phéromone alpha sont les suivantes :

G T A. A G C. T T G. G A T. A A A. A G A.    G C G. C C T. T T G.

Val.   Ser.   Leu.   Asp.   Lys.   Arg.    Ala.   Pro.   Leu

Fin de la région pré-pro de la    Début de la protéine
phéromone alpha    mature

Site de coupure de l'endopep-
tidase de  type cathepsineB :
yscF (produit du gène KEX2).

### EXEMPLE 9 : Sécrétion de la cytokine gro-β par la levure :

1 Transformation de la souche de levure EMY761 par le plasmide pEMR 583 et expression de gro-β par la souche transformée. La souche EMY761 (Mata, leu2, ura3, his3) décrite dans l'exemple 3 a été transformée par le plasmide pEMR 583 pour la prototrophie pour la leucine et selon la technique déjà décrite dans l'exemple 3). Deux transformés ont été retenus, appelés ci-aprés EMY 761 pEMR583 (1) et EMY 761 pEMR 583 (2).
2 Expression en erlenmeyer de l'ADNc de gro-β par les souches EMY 761 pEMR 583 (1) et EMY 761 pEMR 583 (2). Mise en évidence de la protéine dans le milieu de culture sur gel de polyacrylamide sodium dodécyl sulfate (SDS) selon le protocole décrit par LAEMMLI (U.K. LAEMMLI, Nature, 227 [1970] 680-685).

### Culture

a) Une colonie de chacune des souches EMY 761 pEMR 583 (1) et EMY 761 pEMR 583 (2) a été mise en culture dans 50 ml de milieu liquide sans uracile. Ce milieu contient pour 1 1 :
6,7 g de base azotée de levure sans acides aminés (Yeast Nitrogen base without aminoacids de DIFCO)
5,0 g d'hydrolysat de caséine (Casaminoacids de DIFCO)
10 g de glucose.

Après une nuit à 30 ° C sous agitation, les deux cultures ont été centrifugées pendant 10 min à 7 000 tr/min. Les culots ont été repris dans 10 ml d'eau distillée stérile et de nouveau centrifugés pendant 10 min à 7 000 tr/min. L'expression de gro-,8 a été induite en reprenant les cellules dans 50 ml de milieu YNB éthanol-glycérol-galactose. Le milieu YNB éthanol-glycérol-galactose contient pour 1 1 :
6,7 g de base azotée de levure sans acides aminés (Yeast Nitrogen base without Aminoacids de DIFCO).
5,0 g d'hydrolysat de caséine (casaminoacids de DIFCO)
30 g de glycérol
30 g de galactose
10 ml d'éthanol.

Les cultures ont été replacées à 30 ° C sous agitation pendant 24 h.

b) Souche témoin :
La souche EMY 761 non transformée, c'est-à-dire sans plasmide, a été cultivée comme ci-dessus. Elle a subi d'une part la préculture dans 50 ml de milieu liquide sans uracile auquel on a rajouté de l'uracile (20 µg/ml) et, d'autre part, l'induction dans 50 ml de milieu YNB éthanol-glycérol-galactose auquel on a rajouté de l'uracile (20 ug/ml).

### Préparation des échantillons :

Les cellules cultivées en 1 a) et 1 b) ont été centrifugées pendant 20 min à 10 000 tr/min et le surnageant a été recueilli. A 10 ml de surnageant, 5 ml d'acide trichloroacétique à 50 % contenant 2 mg/ml de déoxycholate ont été rajoutés.

Le mélange a été mis à + 4 _{°} C pendant 30 min, puis centrifugé pendant 30 min à 10 000 tr/min. Le culot a été repris dans environ 1 ml d'acétone froid (+4°C) et de nouveau centrifugé 30 min à 10 000 tr/min. Le culot, après avoir été séché, est repris dans environ 20 µl d'un tampon dénommé tampon de charge constitué de Tris-HCI 0,125 M pH 6,8 SDS 4 %, bleu de bromophénol 0,002 %, glycérol 20 %, β-mercaptoéthanol 10 % (selon protocole décrit par LAEMMLI [1970]). Le culot est solubilisé par ébullition pendant 15 min puis neutralisé en ajoutant de la soude 10 N jusqu'à ce que le bleu de bromophénol vire au bleu.

Les échantillons sont déposés sur gel de polyacrylamide/SDS :

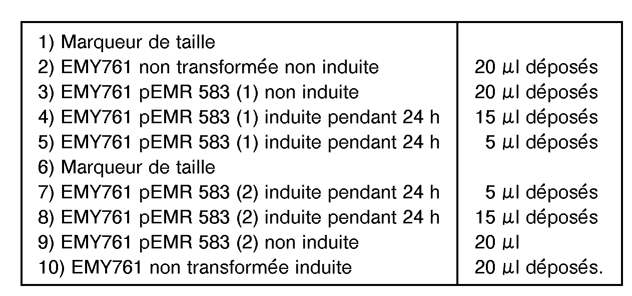

### Après électrophorèse les protéines sont colorées au bleu de Coommassie

### RESULTATS :

L'analyse du gel obtenu montre qu'une protéine surnuméraire de poids moléculaire apparent d'environ 8 kDa est produite par les souches EMY 761 pEMR 583 (1) (pistes 4 et 5) et EMY 761 pEMR 583 (2) (pistes 7 et 8) et n'est pas produite dans les surnageants de culture de la souche EMY 761 non transformée (pistes 2 et 10). Il apparaît également que la synthèse de cette protéine surnuméraire est liée à l'induction du promoteur par croissance sur éthanol-glycérol-galactose (bandes absentes dans les pistes 3 et 9).

L'analyse de la séquence aminoterminale de cette protéine purifiée par HPLC a permis de vérifier qu'il s'agissait de la protéine mature gro-,8 décrite par Tekamp-Olson et al, référence ci-dessus.

Il apparaît donc que la cytokine gro-,8 peut être sécrétée sous le contrôle du promoteur de l'invention.

La souche EMY 761 pEMR 583 (1) a été déposée à la Collection Nationale de Culture de Microorganismes CNCM, Institut Pasteur, France sous le n ° l - 1021.

### EXEMPLE 10 : Construction d'un vecteur d'expression et de sécrétion de l'IL-8 dans la levure : le plasmide pEMR 611, porteur du promoteur artificiel de l'invention :

Un deuxième exemple de protéine sécrétée dont l'expression peut être régulée par le promoteur de l'invention est la cytokine humaine IL-8. Cette cytokine d'environ 8 000 Da, produite par les monocytes, a été décrite par plusieurs équipes : Yoshimura et al (1987) J. Immunol., 139, 788-793, Shrôder et al. (1987), J. Immunol., 139, 3474-3483 et Walz, et al (1987) Biochem-Biophys. Res. Commun, 149, 755-761). L'IL-8 agit comme chimioattractant des neutrophiles. L'IL-8 présente de remarquables similitudes de structure avec la β-thromboglobine (Van Damme et al. (1989) Eur. J. Biochem 181, 337-344). La cytokine IL-8 existe sous plusieurs formes, différant entre elles par leur extrémité NH₂-terminale. La forme majoritaire est composée de 72 aminoacides mais 5 autres formes mineures sont également produites, parmi lesquelles 3 présentent une extrémité NH₂-terminale tronquée par rapport à la forme majoritaire et 2 présentent une extension de respectivement 5 et 1 aminoacides par rapport à cette forme majoritaire.

L'ADNc de l'IL-8 a été cloné et séquencé selon la méthode décrite par Matsushima et al. 1988 J.exp.Med.167, 1883-1993. La séquence de cet ADNc comporte un site unique Hind III (5'-AAGCTT) qui recouvre les 42 et 43e codons de la partie mature (forme 72 aa : Cf. Matsushima et al, réf. citée supra).

D'autre part, le clone de l'ADNc de l'IL-8 utilisé dans ce clonage présente un site BamHl unique flanquant directement la fin (3') de la queue poly A. Le fragment BamHI-Hind III portant l'extrémité 3' de l'ADNc de l'IL-8 a été purifié.

Un vecteur de clonage a été préparé de la manière suivante :
le plasmide pEMR 583 décrit dans l'exemple 8 a été digéré par Hind III et BamHl.

Cette double digestion libère 5 fragments :
le fragment le plus lourd, Hind lll-BamHl correspond au vecteur de clonage (environ 7760 paires de base). Les 4 autres, Hind III-Hind III (de taille de 169 paires de base, 92 paires de base, 529 paires de base et 187 paires de base) correspondent à la séquence de l'ADNc de gro-β. Le site Hind III du vecteur de clonage est localisé un peu en amont du site d'insertion à la fin de la séquence pro de la phéromone alpha (et recouvre la séquence du codon - sérine 81 - du précurseur). Le site BamHl est localisé en amont du terminateur du PGK.

L'ADN du fragment Hind lll-BamHl a été purifié. Le fragment Hind lll-BamHl comportant la partie 3' de la séquence de l'ADNc a été ligué avec un fragment d'ADN synthétique Hind III-Hind III. La séquence de ce fragment, donnée ci-après, est destinée à reconstituer la séquence de la forme mature majoritaire de l'IL-8 (72 aminoacides) précédée de la séquence du site de clivage (Ser-Leu-Asp-Lys-Arg). Le nouveau fragment Hind lll-BamHl a été ligué avec le fragment Hind lll-BamHl correspondant au vecteur de clonage. La séquence du fragment synthétique Hind III-Hind III est la suivante :

5'-AGCTTGGATAAAAGATCTGCTAAGGAATTGAGATGTCAATGTATCAAGACTTACTCTAAGCCATTCC
ACCTATTTTCTAGACGATTCCTTAACTCTACAGTTACATAGTTCTGAATGAGATTCGGTAAGG

ACCCAAAGTTCATCAAGGAATTGAGAGTTATCGAATCTGGTCCACACTGTGCTAACACTGAAATTAT
TGGGTTTCAAGTAGTTCCTTAACTCTCAATAGCTTAGACCAGGTGTGACACGATTGTGACTTTAATA

CGTTA
GCAATTCGA-5'

Le plasmide ainsi obtenu, porteur de l'ADNc de l'IL-8 précédée de la séquence pré-pro de la phéromone alpha et du promoteur de l'invention, dont la séquence a été précisée à l'exemple 2, est appelé pEMR 611.

### EXEMPLE 11 : Transformation de la souche de levure EMY 761 par le plasmide pEMR 611 et sécrétion de l'IL-8.

La souche EMY 761 (Mata, ura3, leu2, his3), décrite dans l'exemple 3, a été transformée en souche (leu+) par l'ADN du plasmide pEMR 611 selon la technique déjà décrite. Parmi les colonies (leu+) obtenues, une a été prélevée au hasard et cultivée pour l'étude de la sécrétion de l'IL-8. Cette souche est appelée par la suite EMY 761 pEMR 611.

Le protocole pour l'analyse des protéines sécrétées par la levure est celui exposé dans l'exemple 9. Les protéines sécrétées par EMY 761 pEMR 611 ont été comparées à celles sécrétées par EMY 761. On a ainsi mis en évidence une bande majeure surnuméraire correspondant à une protéine de 8 000 Da sécrétée par les cellules EMY 761 pEMR 611 induites au galactose. Cette protéine est reconnue spécifiquement par des anticorps de lapin dirigés contre l'IL-8 humaine (fournis par la société Endogen : Anti human IL-8 polyvalent P801), selon les résultats de l'analyse en "Western blot", méthode bien connue de l'homme de l'art et dont le protocole est donné en détail dans l'exemple 4.

Le plasmide d'expression pEMR 611 permet donc l'expression à haut niveau de l'IL-8 dans les levures transformées. Cette expression est sous le contrôle du promoteur artificiel de l'invention.

La souche EMY 761 pEMR 611 a été déposée à la Collection Nationale de Cultures de Microorganismes CNCM, Institut Pasteur, France, sous le n _{°} I - 1023.

### EXEMPLE 12 : Construction d'un vecteur d'expression en sécrétion de l'hirudine : le plasmide pEMR576, porteur du promoteur artificiel de l'invention :

Naturellement produite par la sangsue Hirudo, l'hirudine est un inhibiteur très spécifique et très efficace de la thrombine. Un certain nombre de variants ont été identifiés et désignés par HV₁ , HV₂, HV₃ (Dodt J. et al (1986) FEBS Lett. 202, 373, 377). Par la suite, certains de ces variants naturels ainsi que d'autres analogues ont été préparés par génie génétique dans diverses cellules hôtes. L'exemple concerne le variant rHV₂-Lys47 décrit dans la publication de brevet EP-A-0 273 800. Plus particulièrement, la séquence exprimée code pour un précurseur de ce variant rHV₂-Lys47,caractérisé en ce qu'il contient une séquence signal: Met - Arg - Phe - Ser - Thr - Thr - Val - Ala - Thr - Ala - Ala - Tyr - Ala - Leu - Phe - Phe - Thr - Ala - Ser - Gln - Val - Ser - Ala précédant directement le début de la séquence de l'hirudine mature.

La construction et la structure de ce précurseur sont décrits dans la publication de brevet FR 2 646 437. L'expression de ce précurseur permet la libération du variant rHV₂-Lys47 dans le surnageant de culture des cellules transformées.

Le plasmide pTG3867, dont la construction et la description sont détaillées dans la demande de brevet FR 2 646 437, est un vecteur de sécrétion pour l'hirudine. Dans cette construction, l'hirudine est synthétisée sous forme d'un précurseur contenant une séquence signal. L'hirudine est placée derrière le promoteur du gène MFa1 qui est constitutif dans les souches de levure de type conjugant alpha.

### Construction du plasmide pEMR 547

Le plasmide pEMR547 dérive du plasmide pEMR515 (cf. exemple 2) par délétion de la petite séquence provenant du plasmide 2 µ, et localisée entre la fin du gène LEU2-d et le site EcoRI bordant la séquence du plasmide pBR322. Le plasmide pEMR515 a été digéré partiellement par BspMl et totalement par EcoRI. Le grand fragment BspMI-EcoRI correspondant au plasmide pEMR515 délété de la partie 3' de LEU2 et la petite séquence attenante du 2 µ a été ligué avec une séquence synthétique BspMI-EcoRI ° destinée à reconstituer la région 3' du gène LEU2-d.

Cette séquence synthétique est la suivante :

EcoRI°
AATTGCCCGGGACGTCTTATGTACAAATATCATAAAAAAAGAGAATCTTTTT
+---------+---------+---------+---------+---------
CGGGCCCTGCAGAATACATGTTTATAGTATTTTTTTCTCTTAGAAAAA

AAGCAAGGATTTTCTTAACTTCTTCGGCGACAGCATCACCGACTTCGGTGGTACTGTTGG
+---------+---------+---------+---------+---------+---------
TTCGTTCCTAAAAGAATTGAAGAAGCCGCTGTCGTAGTGGCTGAAGCCACCATGACAACC

B
s
p
M
I
AACCACCTAAATCACCAGTTCTGATACCTGCATCC
+---------+---------+---------+--------
TTGGTGGATTTAGTGGTCAAGACTATGGACGTAGGTTTT

Le plasmide reconstitué est appelé pEMR547.

### Construction du plasmide pEMR568

Le plasmide pEMR568 dérive de pEMR547 de la manière suivante :
L'ADN du plasmide pEMR547 a été digéré par Mlul et Nhel. Cette double digestion permet de linéariser le plasmide pEMR547 (6,6 kb), les 2 sites Mlul et Nhel étant situés à quelques paires de base l'un de l'autre. Entre ces 2 sites, on a rentré un oligonucléotide synthétique double brin de séquence :

CTAGCGAGCTCAAGCTTA
GCTCGAGTTCGAATGCGC

Le plasmide résultant est appelé pEMR568.

### Construction du plasmide pEMR576, plasmide d'expression de l'hirudine.

La séquence du variant rHV₂-Lys47 de l'hirudine a été obtenue à partir du plasmide pTG3867, dont la construction et la description sont détaillées dans la demande de brevet FR 2 646 437. Dans cette construction, l'hirudine est synthétisée sous forme d'un précurseur composé d'un peptide signal de 23 aminoacides (y compris la méthionine correspondant au codon d'initiation). L'ARN messager codant pour le précurseur est transcrit à l'aide du promoteur MFa1 qui est constitutif dans les souches de levure de type conjugant alpha.

La double digestion Accl-Sall du plasmide pTG3867 libère plusieurs fragments dont le plus court, d'environ 200 paires de base, est facilement purifiable sur gel d'agarose 2 %. Le site Accl qui borde ce fragment est localisé quelques paires de base du début de la séquence mature selon la séquence :

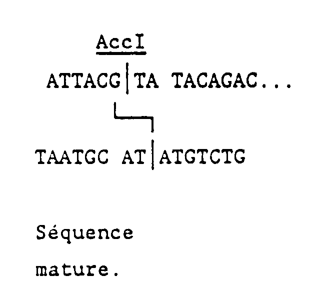
AccI
ATTACG|TA TACAGAC...
TAATGC AT|ATGTCTG

Séquence
mature.

Le site Sall est localisé en aval du codon stop de la séquence codante de l'hirudine.

Le fragment Accl-Sall de 200 paires de base porte l'information pour la plus grande partie de la séquence mature de l'hirudine. Le complément d'information (séquence signal et début de la séquence mature) est fourni par une séquence synthétique d'environ 90 nucléotides, précisée ci-après :

CGATATACACAATGCGTTTCTCTACTACAGTCGCTACTGCAGCTACTGCGCTATTTTTCACAGCCTCC
TATATGTGTTACGCAAAGAGATGATGTCAGCGATGACGTCGATGACGCGATAAAAAGTGTCGGAGG

CCAAGTTTCAGCTATTACGT
GGTTCAAAGTCGATAATGCATA

Le vecteur pEMR468 a été linéarisé par Sall et digéré partiellement par Clal. Le fragment Clal-Sall d'environ 5,6 kb, correspondant à ce vecteur délété de la séquence de l'urate oxydase, a été ligué avec le fragment Accl-Sall, d'environ 200 paires de base, qui porte l'information pour la séquence de l'hirudine mature ; et avec la petite séquence synthétique Clal-Accl destinée à reconstituer la séquence du précurseur de l'hirudine. Le plasmide résultant est appelé pEMR576.

### EXEMPLE 13 : Sécrétion de l'hirudine

### 1) Transformation de la souche EMY761 par le plasmide pEMR576.

La souche EMY761 (Mata, ura3, his3, leu2) a été transformée en souche (leu+) par le plasmide pEMR576, suivant la technique déjà décrite.

Un transformé a été isolé, ci-après appelé EMY761 pEMR576.

### 2) Expression de l'hirudine.

Comme témoin négatif, on a construit une souche (leu+) dérivée de EMY761 et appelée ci-après EMY761 (leu+). La technique utilisée est décrite en détail dans la demande de brevet FR 2 646 437. Les souches EMY761 pEMR576 et EMY761 (leu+) ont été cultivées en parallèle comme décrit ci-après :
Les précultures se font dans du milieu de composition :
Yeast Nitrogen Base (Difco) 0,7 %, histidine 50 ug/ml et uracile 50 µg/ml. Au bout de 24 h, on ensemence les cultures avec 10⁵ cellules dans du milieu de composition Yeast Nitrogen Base (Difco) 0,7 %, éthanol 1 %, casaminoacides 0,5 %, uracile 100 µg/ml, glycérol 3 % et galactose 1 %.

Au bout de 72 h de culture dans ce dernier milieu, on sépare par filtration sur 0,2 µ le surnageant des cellules. L'activité inhibitrice du surnageant sur la thrombine est mesurée en utilisant le test colorimétrique décrit dans FR 2 646 437 (activité protéolytique de la thrombine sur un substrat synthétique, le chromozy- me TH commercialisé par Boehringer Mannheim).

Le tableau ci-dessous présente les résultats des dosages en ug d'hirudine par ml de surnageant, à une densité optique de 1, soit 0,3 x 10⁷ cellules/ml.

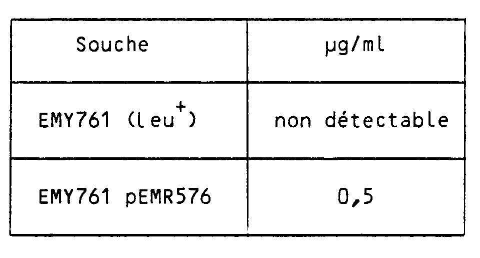

Il apparaît donc que l'hirudine peut être sécrétée sous le contrôle du promoteur de l'invention.

La souche EMY761 pEMR576 a été déposée auprès de la Collection Nationale de Culture de Microorganismes CNCM - Institut Pasteur - France, sous le n ° l - 1022.

## Revendications

1. Promoteur artificiel pour l'expression de protéines dans la levure, caractérisé en ce qu'il comporte :
- une sous-séquence en amont de l'élément TATA de la séquence du promoteur du gène GAL7 de Saccharomyces cerevisiae, qui comporte les séquences d'activation amont UAS1 et UAS2 ;
- une sous-séquence de la séquence d'un promoteur ADH₂ comportant l'élément TATA et la région d'initiation de la transcription.

2. promoteur selon la revendication 1, caractérisé en ce que la sous-séquence de la séquence du promoteur GAL7 de Saccharomyces cerevisiae est la séquence suivante ou l'une des sous-séquences de celle-ci :

M
l
u
I
CGCGTCTATACTTCGGAGCACTGTTGAGCGAAGGCTCATTAGATATATTTTCTGTCAT
AGATATGAAGCCTCGTGACAACTCGCTTCCGAGTAATCTATATAAAAGACAGTA
TTTCCTTAACCCAAAAATAAGGGAGAGGGTCCAAAAAGCGCTCGGACAACTGTTGACCGT
AAAGGAATTGGGTTTTTATTCCCTCTCCCAGGTTTTTCGCGAGCCTGTTGACAACTGGCA
GATCCGAAGGACTGGCTATACAGTGTTCACAAAATAGCCAAGCTGAAAATAATGTGTAGC
CTAGGCTTCCTGACCGATATGTCACAAGTGTTTTATCGGTTCGACTTTTATTACACATCG
S
p
h
I
CTTTAGCTATGTTCAGTTAGTTTGGCATG
GAAATCGATACAAGTCAATCAAACC

3. Promoteur selon l'une des revendications 1 et 2, caractérisé en ce que la sous-séquence de la séquence d'un promoteur ADH₂ comprenant l'élément TATA et la région d'initiation est la séquence ci-après ou l'une des sous-séquences de celle-ci :

CCTATCACATATAAATAGA
βTACGGATAGTGTATATTTATCT

GTGCCAGTAGCGACTTTTTTCACACTCGAGATACTCTTACTACTGCTCTCTTGTTGTTTT
CACGGTCATCGCTGAAAAAAGTGTGAGCTCTATGAGAATGATGACGAGAGAACAACAAAA

TATCACTTCTTGTTTCTTCTTGGTAAATAGAA[?]ATCAAGCTACAAAAAGCATACAATCAA
ATAGTGAAGAACAAAGAAGAACCATTTATCTTATAGTTCGATGTTTTTCGTATGTTAGTT

C
l
a
I
CTATCAACTATTAACTATA[?]
GATAGTTGATAATTGATATAGC

4. Promoteur selon l'une des revendications 1 à 3, caractérisé en ce qu'il comporte la séquence suivante :

M
l
u
I
CGCGTCTATACTTCGGAGCACTGTTGAGCGAAGGCTCATTAGATATATTTTCTGTCAT
--+---------+---------+---------+---------+---------+-----
    AGATATGAAGCCTCGTGACAACTCGCTTCCGAGTAATCTATATAAAAGACAGTA

TTTCCTTAACCCAAAAATAAGGGAGAGGGTCCAAAAAGCGCTCGGACAACTGTTGACCGT
----+---------+---------+---------+---------+---------+-----
AAAGGAATTGGGTTTTTATTCCCTCTCCCAGGTTTTTCGCGAGCCTGTTGACAACTGGCA

GATCCGAAGGACTGGCTATACAGTGTTCACAAAATAGCCAAGCTGAAAATAATGTGTAGC
----+---------+---------+---------+---------+---------+-----
CTAGGCTTCCTGACCGATATGTCACAAGTGTTTTATCGGTTCGACTTTTATTACACATCG

S
p
h
I
CTTTAGCTATGTTCAGTTAGTTTGGCATGCCTATCACATATAAATAGA
----+---------+---------+---------+-------------
GAAATCGATACAAGTCAATCAAACCGTACGGATAGTGTATATTTATCT

GTGCCAGTAGCGACTTTTTCACACTCGAGATACTCTTACTACTGCTCTCTTGTTGTTTT
---+---------+---------+---------+---------+---------+------
CACGGTCATCGCTGAAAAAGTGTGAGCTCTATGAGAATGATGACGAGAGAACAACAAAA

TATCACTTCTTGTTTCTTCTTGGTAAATAGAATATCAAGCTACAAAAAGCATACAATCAA
---+---------+---------+---------+---------+---------+------
ATAGTGAAGAACAAAGAAGAACCATTTATCTTATAGTTCGATGTTTTTCGTATGTTAGTT

C
l
a
I
CTATCAACTATTAACTATAT
---+---------+------
GATAGTTGATAATTGATATAGC

5. Vecteur d'expression pour la levure portant un gène d'intérêt avec les moyens nécessaires à son expression, sa réplication et la sélection des cellules transformées, caractérisé en ce que ce gène d'intérêt est sous le contrôle du promoteur selon l'une des revendications 1 à 4.

6. Vecteur d'expression selon la revendication 5, caractérisé en ce que le gène d'intérêt est un gène recombinant codant pour l'urate oxydase.

7. Vecteur d'expression selon la revendication 5, caractérisé en ce que le gène d'intérêt est un gène recombinant codant pour une cytokine humaine.

8. Vecteur d'expression selon la revendication 5, caractérisé en ce que le gène d'intérêt est un gène recombinant codant pour l'hirudine.

9. Souche de Saccharomyces cerevisiae, caractérisée en ce qu'elle est transformée par un vecteur d'expression selon l'une des revendications 5 à 8.

10. Procédé de production d'une protéine d'intérêt, caractérisé en ce qu'il comprend la culture d'une souche de Saccharomyces cerevisiae selon la revendicaton 9 en présence de galactose.

## Claims

1. Artificial promoter for the expression of proteins in yeast, characterized in that it comprises:
- a sub-sequence upstream from the TATA component of the sequence of the promoter of the GAL7 gene of Saccharomyces cerevisiae, which comprises the upstream activation sequences UAS1 and UAS2;
- a sub-sequence of the sequence of an ADH₂ promoter comprising the TATA component and the transcription initiation region.

2. Promoter according to claim 1, characterized in that the sub-sequence of the sequence of the GAL7 promoter of Saccharomyces cerevisiae is the following sequence or a sub-sequence thereof:

M
l
u
I

CGCGTCTATACTTCGGAGCACTGTTGAGCGAAGGCTCATTAGATATATTTTCTGTCAT
--+---------+---------+---------+---------+---------+-----
    AGATATGAAGCCTCGTGACAACTCGCTTCCGAGTAATCTATATAAAAGACAGTA

TTTCCTTAACCCAAAAATAAGGGAGAGGGTCCAAAAAGCGCTCGGACAACTGTTGACCGT
----+---------+---------+---------+---------+---------+-----
AAAGGAATTGGGTTTTTATTCCCTCTCCCAGGTTTTTCGCGAGCCTGTTGACAACTGGCA

GATCCGAAGGACTGGCTATACAGTGTTCACAAAATAGCCAAGCTGAAAATAATGTGTAGC
----+---------+---------+---------+---------+---------+-----
CTAGGCTTCCTGACCGATATGTCACAAGTGTTTTATCGGTTCGACTTTTATTACACATCG

                            S
                            p
                            h
                            I
CTTTAGCTATGTTCAGTTAGTTTGGCATG
----+---------+---------+----
GAAATCGATACAAGTCAATCAAACC

3. Promoter according to one of claims 1 and 2, characterized in that the sub-sequence of the sequence of an ADH₂ promoter comprising the TATA component and the initiation region is the following sequence or a sub-sequence thereof:

CCTATCACATATAAATAGA
-------------------
5TACGGATAGTGTATATTTATCT

GTGCCAGTAGCGACTTTTTTCACACTCGAGATACTCTTACTACTGCTCTCTTGTTGTTTT
------------------------------------------------------------
CACGGTCATCGCTGAAAAAAGTGTGAGCTCTATGAGAATGATGACGAGAGAACAACAAAA

TATCACTTCTTGTTTCTTCTTGGTAAATAGAA[?]ATCAAGCTACAAAAAGCATACAATCAA
------------------------------------------------------------
ATAGTGAAGAACAAAGAAGAACCATTTATCTTATAGTTCGATGTTTTTCGTATGTTAGTT

C
l
a
I
CTATCAACTATTAACTATA[?]
--------------------
GATAGTTGATAATTGATATAGC

4. Promoter according to one of claims 1 to 3, characterized in that it comprises the following sequence:

M
l
u
I
CGCGTCTATACTTCGGAGCACTGTTGAGCGAAGGCTCATTAGATATATTTTCTGTCAT
---+---------+---------+---------+---------+---------+-----
AGATATGAAGCCTCGTGACAACTCGCTTCCGAGTAATCTATATAAAAGACAGTA

TTTCCTTAACCCAAAAATAAGGGAGAGGGTCCAAAAAGCGCTCGGACAACTGTTGACCGT
----+---------+---------+---------+---------+---------+-----
AAAGGAATTGGGTTTTTATTCCCTCTCCCAGGTTTTTCGCGAGCCTGTTGACAACTGGCA

GATCCGAAGGACTGGCTATACAGTGTTCACAAAATAGCCAAGCTGAAAATAATGTGTAGC
----+---------+---------+---------+---------+---------+-----
CTAGGCTTCCTGACCGATATGTCACAAGTGTTTTATCGGTTCGACTTTTATTACACATCG

S
p
h
I
CTTTAGCTATGTTCAGTTAGTTTGGCATGCCTATCACATATAAATAGA
----+---------+---------+---------+---------+---
GAAATCGATACAAGTCAATCAAACCGTACGGATAGTGTATATTTATCT

GTGCCAGTAGCGACTTTTTTCACACTCGAGATACTCTTACTACTGCTCTCTTGTTGTTTT
----+---------+---------+---------+---------+---------+-----
CACGGTCATCGCTGAAAAAAGTGTGAGCTCTATGAGAATGATGACGAGAGAACAACAAAA

TATCACTTCTTGTTTCTTCTTGGTAAATAGAATATCAAGCTACAAAAAGCATACAATCAA
----+---------+---------+---------+---------+---------+-----
ATAGTGAAGAACAAAGAAGAACCATTTATCTTATAGTTCGATGTTTTTCGTATGTTAGTT

C
l
a
I
[?]TATCAACTATTAACTATAT
----+---------+-----
GATAGTTGATAATTGATATAGC

5. Expression vector for yeast, carrying a gene of interest with the means necessary for its expression, its replica and the selection of the transformed cells, characterized in that this gene of interest is under the control of the promoter according to one of claims 1 to 4.

6. Expression vector according to claim 5, characterized in that the gene of interest is a recombinant gene coding for urate oxidase.

7. Expression vector according to claim 5, characterized in that the gene of interest is a recombinant gene coding for a human cytokin.

8. Expression vector according to claim 5, characterized in that the gene of interest is a recombinant gene coding for Hirudin.

9. Strain of Saccharomyces cerevisiae, characterized in that it is transformed by an expression vector according to one of claims 5 to 8.

10. Method of producing a protein of interest, characterized in that it comprises culturing a strain of Saccharomyces cerevisiae according to claim 9, in the presence of galactose.

## Patentansprüche

1. Künstlicher Promotor zur Expression von Proteinen in Hefe,
dadurch gekennzeichnet, daß er enthält:
- eine Teilsequenz stromaufwärts der TATA-Box der Promotorsequenz des GAL7-Gens von Saccharomyces cerevisiae, die die Stromaufwärts-Aktivierungssequenzen UAS1 und UAS2 enthält,
- eine Teilsequenz der Sequenz eines ADH₂-Promotors, die die TATA-Box und die Initiationsregion der Transkription enthält.

2. Promotor nach Anspruch 1,
dadurch gekennzeichnet, daß
die Teilsequenz der GAL7-Promotorsequenz von Saccharomyces cerevisiae die folgende Sequenz oder eine der Teilsequenzen dieser Sequenz ist:

MluI

CGCGTCTATACTTCGGAGCACTGTTGAGCGAAGGCTCATTAGATATATTTTCTGTCAT
--+---------+---------+---------+---------+---------+-----
    AGATATGAAGCCTCGTGACAACTCGCTTCCGAGTAATCTATATAAAAGACAGTA

TTTCCTTAACCCAAAAATAAGGGAGAGGGTCCAAAAAGCGCTCGGACAACTGTTGACCGT
----+---------+---------+---------+---------+---------+-----
AAAGGAATTGGGTTTTTATTCCCTCTCCCAGGTTTTTCGCGAGCCTGTTGACAACTGGCA

GATCCGAAGGACTGGCTATACAGTGTTCACAAAATAGCCAAGCTGAAAATAATGTGTAGC
----+---------+---------+---------+---------+---------+-----
CTAGGCTTCCTGACCGATATGTCACAAGTGTTTTATCGGTTCGACTTTTATTACACATCG

SphI
CTTTAGCTATGTTCAGTTAGTTTGGCATG
----+---------+---------+----
GAAATCGATACAAGTCAATCAAACC

3. Promotor nach einem der Ansprüche 1 und 2,
dadurch gekennzeichnet, daß
die Teilsequenz der Sequenz eines ADH₂-Promotors, die die TATA-Box und die Initiationsregion enthält, die folgende Sequenz oder eine der Teilsequenzen dieser Sequenz ist:

CCTATCACATATAAATAGA
.....................
[?]TACGGATAGTGTATATTTATCT

GTGCCAGTAGCGACTTTTTTCACACTCGAGATACTCTTACTACTGCTCTCTTGTTGTTTT
............................................................
CACGGTCATCGCTGAAAAAAGTGTGAGCTCTATGAGAATGATGACGAGAGAACAACAAAA

TATCACTTCTTGTTTCTTCTTGGTAAATAGAA[?]ATCAAGCTACAAAAAGCATACAATCAA
............................................................
ATAGTGAAGAACAAAGAAGAACCATTTATCTTATAGTTCGATGTTTTTCGTATGTTAGTT

C
l
a
I
CTATCAACTATTAACTATA[?]
....................
GATAGTTGATAATTGATATAGC

4. Promotor nach einem der Ansprüche 1 bis 3,
dadurch gekennzeichnet, daß
er die folgende Sequenz enthält:

MluI

CGCGTCTATACTTCGGAGCACTGTTGAGCGAAGGCTCATTAGATATATTTTCTGTCAT
---+---------+---------+---------+---------+---------+-----
    AGATATGAAGCCTCGTGACAACTCGCTTCCGAGTAATCTATATAAAAGACAGTA

TTTCCTTAACCCAAAAATAAGGGAGAGGGTCCAAAAAGCGCTCGGACAACTGTTGACCGT
---+---------+---------+---------+---------+---------+------
AAAGGAATTGGGTTTTTATTCCCTCTCCCAGGTTTTTCGCGAGCCTGTTGACAACTGGCA

GATCCGAAGGACTGGCTATACAGTGTTCACAAAATAGCCAAGCTGAAAATAATGTGTAGC
---+---------+---------+---------+---------+---------+------
CTAGGCTTCCTGACCGATATGTCACAAGTGTTTTATCGGTTCGACTTTTATTACACATCG

SphI

CTTTAGCTATGTTCAGTTAGTTTGGCATGCCTATCACATATAAATAGA
---+---------+---------+---------+---------+----
GAAATCGATACAAGTCAATCAAACCGTACGGATAGTGTATATTTATCT

GTGCCAGTAGCGACTTTTTTCACACTCGAGATACTCTTACTACTGCTCTCTTGTTGTTTT
---+---------+---------+---------+---------+---------+------
CACGGTCATCGCTGAAAAAAGTGTGAGCTCTATGAGAATGATGACGAGAGAACAACAAAA

TATCACTTCTTGTTTCTTCTTGGTAAATAGAATATCAAGCTACAAAAAGCATACAATCAA
---+---------+---------+---------+---------+---------+------
ATAGTGAAGAACAAAGAAGAACCATTTATCTTATAGTTCGATGTTTTTCGTATGTTAGTT

ClaI

CTATCAACTATTAACTATAT
---+---------+------
GATAGTTGATAATTGATATAGC

5. Expressionsvektor für Hefe, der ein interessierendes Gen trägt sowie Mitteln aufweist, die für die Expression, die Replikation und die Selektion transformierter Zellen erforderlich sind,
dadurch gekennzeichnet, daß
das interessierende Gen unter der Kontrolle des Promotors nach einem der Ansprüche 1 bis 4 steht.

6. Expressionsvektor nach Anspruch 5,
dadurch gekennzeichnet, daß
das interessierende Gen ein rekombinantes Gen ist, das die Uratoxidase codiert.

7. Expressionsvektor nach Anspruch 5,
dadurch gekennzeichnet, daß
das interessierende Gen ein rekombinantes Gen ist, das ein menschliches Cytokin codiert.

8. Expressionsvektor nach Anspruch 5,
dadurch gekennzeichnet, daß
das interessierende Gen ein rekombinantes Gen ist, das Hirudin codiert.

9. Saccharomyces-cerevisiae-Stamm,
dadurch gekennzeichnet, daß
er durch einen Expressionsvektor nach einem der Ansprüche 5 bis 8 transformiert ist.

10. Verfahren zur Herstellung eines interessierenden Proteins,
dadurch gekennzeichnet, daß
es die Kultivierung eines Saccharomyces-cerevisiae-Stamms nach Anspruch 9 in Gegenwart von Galactose umfaßt.
